# EUROPÄISCHE PATENTSCHRIFT

(11) **EP 3 773 127 B1**
(45) Veröffentlichungstag und Bekanntmachung des Hinweises auf die Patenterteilung: **11.09.2024**
(21) Anmeldenummer: 19714192.2
(22) Anmeldetag: 26.03.2019
(51) Int. Cl.: A61B 1/00, A61B 1/07, F21V 8/00, G02B 23/24, G02B 23/26, G02B 6/26, G02B 6/04, G02B 6/32, G02B 6/36

(54) **LICHT- ODER BILDLEITKOMPONENTEN FÜR EINWEG-ENDOSKOPE**
LIGHT GUIDE OR IMAGE GUIDE COMPONENTS FOR SINGLE-USE ENDOSCOPES
ÉLÉMENT CONDUCTEUR DE LUMIÈRE OU D'IMAGE POUR ENDOSCOPE JETABLE

(30) Priorität: 29.03.2018 DE 102018107523
(43) Veröffentlichungstag der Anmeldung: 17.02.2021
(73) Patentinhaber: SCHOTT AG, 55122 Mainz (DE)
(72) Erfinder: SCHULTHEIS, Bernd, 55270 Schwabenheim (DE); BLEISINGER, Björn, 55499 Riesweiler (DE); DIETRICH, Andreas, 55452 Guldental (DE); KAPPEL, Markus, 55595 Roxheim (DE); RUSSERT, Hubertus, 55270 Jugenheim (DE); CRAMER, Martin, 65187 Wiesbaden (DE); WEINGÄRTNER, Thomas, 55435 Gau-Algesheim (DE)
(74) Vertreter: Blumbach · Zinngrebe Patentanwälte PartG mbB
(86) Internationale Anmeldenummer: PCT/EP2019/057616
(87) Internationale Veröffentlichungsnummer: WO 2019/185645

(56) Entgegenhaltungen:
- EP-A1- 1 890 173
- WO-A1-2015/168247
- DE-A1- 102008 044 938
- DE-A1- 102014 208 756
- US-A1- 2012 289 779
- US-A1- 2014 350 343
- US-A1- 2015 374 217
- US-A1- 2016 334 616

## Beschreibung

Die Erfindung ist definiert durch Anspruch 1 und betrifft ein Diagnose-, Operations- und/ oder Therapiegerät zur Einbringung in den menschlichen oder tierischen Körper oder zur in-vitro Untersuchung von menschlichen oder tierischen Blutproben oder sonstigen Körperzellen, insbesondere ein Endoskop oder ein Einweg-Endoskop, beinhaltend zumindest einen Beleuchtungslichtleiter und/ oder Bildleiter zur Transmission elektromagnetischer Strahlung, wobei der Beleuchtungslichtleiter oder der Bildleiter jeweils eine proximale Endfläche zur Ein- bzw. Auskopplung von elektromagnetischer Strahlung und eine distale Endfläche zur Aus- bzw. Einkopplung elektromagnetischer Strahlung aufweisen.

Endoskope zur Diagnose, für minimalinvasive Eingriffe oder zur Therapie sind als starre oder flexible Ausführungen bekannt und in der Literatur hinreichend beschrieben. Einweg-Endoskope, oder auch "disposable Endoscopes" genannt, werden heute zunehmend eingesetzt, um insbesondere die Patientensicherheit bei medizintechnische Untersuchungen, Therapien und/ oder minimal invasive Eingriffen zu erhöhen, in dem durch eine Einmal-Verwendung Kontaminationen verhindert werden. Zwar sind bisherige Endoskope dafür konzipiert, dass diese im Sinne der Medizintechnik aufbereitbar, das heißt reinigbar, sterilisierbar und vor allem autoklavierbar, sind.

Trotzdem kann es hier aufgrund falscher Anwendung der Aufbereitung bzw. ungünstigem Design derartiger Geräte vereinzelt vorkommen, dass nicht die erforderliche Keimzahlreduzierung erreicht wird, und damit Keime bei der nächsten Anwendung auf den Patienten übertragen werden können. Dies kann durch den Einsatz von derartigen Einweg-Endoskopen verhindert werden.

Ein weiterer Aspekt für den vermehrten Einsatz von Einweg-Endoskopen stellt auch eine Wirtschaftlichkeitsbetrachtung dar. Insbesondere der ordnungsgemäß und regelmäßig nach jeder Behandlung durchgeführte Aufbereitungsprozess erfordert inzwischen hohe Kosten beim praktizierenden Arzt oder in der Klinik. Zudem sind hohe Investitionen für Reinigungsgeräte, wie Thermodesinfektoren, und Autoklaviergeräten und/ oder Plasma-Sterilisationsgeräte erforderlich, so dass insgesamt der Einsatz derartiger Einweg-Endoskope gerechtfertigt ist.

Ein weiterer Vorteil ergibt sich daraus, dass derartige Einweg-Endoskope zum einen mobil als "hand-held"-Geräte einsetzbar sind und daher auch in der Notfall-Medizin, im Militär-Sanitätseinsatz oder auch in schwer zugänglichen Regionen, zum Beispiel auch bei Katastropheneinsätzen, einsetzbar sind, wo insbesondere keine Aufbereitungsmöglichkeiten zur Verfügung stehen.

Derartige Einweg-Endoskope, "Single-Use"-Endoskope oder "disposable Endoscopes", wie sie in der Literatur beschrieben sind, sind beispielhaft in folgenden Schriften beschrieben:
Die Schrift US 3581738 A1 offenbart ein Einweg-Endoskop, umfassend einen Körper aus synthetischem Harzmaterial mit einer im allgemeinen rohrförmigen Seitenwand, die ein Spekulum bildet, und ein einheitliches längliches Lichtleitelement, das in die Seitenwand eingebettet ist, wobei das Element aus einem Lichtleitmaterial gebildet ist, das mit einem transparenten Material mit einem Brechungsindex, der sich von dem des Lichtleitmaterials unterscheidet, überzogen ist, wobei der Körper aus zwei sich axial vom Endoskop geteilten Paarungshälften gebildet ist, wobei jede Hälfte ein Elementumschließungselement aufweist.

In der Schrift US 4964710 A1 wird ein starres Endoskop beschrieben, welches mit einem Objektivsystem, einer Okularlinse und einer Zwischenrelaislinse ausgestattet. Das Relaissystem ist ein Hybridsystem, das sowohl Kunststoff- als auch Glaselemente verwendet. Die Kunststoffelemente bestehen aus einer gleichmäßigen Mehrzahl (N) axial ausgerichteter Linsen, die jeweils eine Länge in der gleichen Reihenfolge wie ihr Durchmesser aufweisen. Die Kunststofflinsen sind eine Vielzahl (N minus 1) von axial ausgerichteten Glas-Planzylindern, deren Stirnflächen poliert sind.

Die Schrift EP 1890173 A1 beschreibt eine Methode zur Herstellung eines Lichtleiters, wie er in derartigen Endoskopen einsetzbar ist. Dabei werden eine Vielzahl von optischen Fasern gebündelt und anschließend das Faserbündel an einem Teil eines Mundstücks geschnitten, das an einem Zwischenteil des Faserbündels befestigt ist. So wird das Faserbündel in ein erstes optisches Faserbündel und ein zweites optisches Faserbündel aufgeteilt. Die Teilungsflächen des ersten und zweiten optischen Faserbündels haben die gleichen Eigenschaften und Bedingungen, da die ersten und zweiten optischen Faserbündel aus dem Faserbündel gebildet sind, das durch Bündelung der gleichen optischen Fasern erhalten wird. Das erste optische Faserbündel ist in einem Einführungsabschnitt eines Endoskops montiert und das zweite optische Faserbündel ist in einem flexiblen Schlauch montiert, womit ein erster Lichtleiter in dem Einführungsabschnitt des Endoskops und ein zweiter Lichtleiter in dem flexiblen Schlauch ausgebildet sind. Dadurch entsteht eine trennbare Lichttransmissionsstrecke des Lichtleiters.

Weiterer Stand der Technik ist in den Schriften DE 10 2014 208756 A1, DE 10 2008 044938 A1, US 2015/374217 A1 und US 2012/289779 A1 beschrieben.

Da aufgrund ihrer Einmal-Verwendung derartige Endoskope unter einem hohen Kostendruck stehen, müssen die Baugruppen bzw. Komponenten kostenoptimiert herstellbar sein. Eine der Hauptkomponenten zur Bildgebung und Beleuchtung stellen Beleuchtungslichtleiter oder Bildleiter dar. Diese werden derzeit noch in vergleichsweise aufwendigen Prozessschritten montiert bzw. bearbeitet. Oft sind es komplexe mechanische Komponenten, teils kombiniert mit optischen Elementen, wie Linsen, die diese Licht- bzw. Bildleiter beinhalten, und teils sind es auch aufwendige Bearbeitungsschritte, wie das Schleifen und Polieren der Endfläche, die derzeitige Beleuchtungslichtleiter bzw. Bildleiter vergleichsweise kostenaufwendig machen. Andererseits müssen aber auch noch gewisse lichttechnische Anforderungen beim endoskopischen Einsatz insbesondere in der Medizintechnik berücksichtigt werden. Dies sind neben möglichst verlustfreier Bereitstellung des von einer Lichtquelle bereitgestellten Lichtes an den Untersuchungsort, farbtreue bzw. gezielt farbige Darstellung des Untersuchungsortes auch die Vermeidung unnötige Wärme an den Untersuchungsort einzubringen.

Bei Verwendung von aktiven elektronischen Bauelementen, wie beispielsweise Kamera-Chips und/ oder LED zur Beleuchtung müssen zudem noch Anforderungen hinsichtlich elektrischer Isolation, elektrischer Abschirmung, sowie Patientenableitströmen berücksichtigt werden, die je nach Einsatzgebiet des Endoskops maximale Grenzwerte nicht überschreiten dürfen. So ist beispielsweise bei Anwendungen am Herzen ein max. Ableitstrom von 10 µA gefordert, was einer CF-Klassifizierung entspricht (vergl. EN 60601-1, 3. Ausgabe Tab. 3).

Neben diesen lichttechnischen und elektrischen Anforderungen sind auch noch Anforderungen hinsichtlich der Biokompatibilität zu beachten. Für die Biokompatibilität ist es erforderlich sicherzustellen, dass das Material mit menschlichem Organismus verträglich ist. Für Medizinprodukte, die in Kontakt zum menschlichen Körper kommen können, ist es regulatorisch gefordert, mögliche Interaktionen und unerwünschte Nebenwirkungen zu bestimmen und zu bewerten. Die Wahl der erforderlichen Prüfungen ergibt sich aus der Kontaktart und der Kontaktdauer im menschlichen Körper. Entsprechend der europäischen Richtlinie für Medizinprodukte MDD 93/42 EWG ist diese biologische Beurteilung eines Produktes immer dann notwendig, wenn ein unmittelbarer Kontakt von Werkstoff/Produkt mit dem Patienten besteht.

Die Hauptregelwerke zur biologischen Untersuchung und Beurteilung von Werkstoffen sind die DIN EN ISO 10993 und die Prüfung nach United States Pharmacopeia Class VI (USP Class VI). Obwohl die deutlich umfangreichere ISO 10993 ursprünglich den Test nach USP Class VI ersetzen sollte, wird die USP-Prüfung heute insbesondere sehr häufig zur Beurteilung von biokompatiblen Kunststoffen herangezogen. Dazu werden die für eine invasive Applikation vorgesehenen Materialien zum einen hinsichtlich Ihrer chemischen Verbindung bewertet und zum anderen einem Zytotoxizitätstest unterzogen, bei dem möglichen toxischen Wirkungen auflebende Zellkulturen untersucht werden. Die Anforderungen hierfür sind in der DIN EN ISO 10993, insbesondere in den Teilen -1 und -5 zusammengefasst (DIN EN ISO 10993-1: 2010-04). In USA unterliegt dies den Anforderungen der FDA. Zur DIN EN ISO 10993 korrespondierende Anforderungen sind dort in der USP Class VI hinterlegt.

Weiterhin kommt der Auslegung der Endoskope als Einweg-Endoskope zu Gute, dass die als Aufbereitungsmethoden bekannten Reinigungs-/ Desinfektionsverfahren mit stark basischen Lösungen und die Sterilisation mittels Autoklavieren bei Temperatur bis zu 135° C und typischen Dampfdrücken von etwa 3 bar bei der Materialauswahl nicht in diesem Maße berücksichtigt werden müssen, was insbesondere auch eine kostengünstigere Materialauswahl erlaubt. Lediglich sind RoHS- als auch REACH-Bestimmungen bei den Materialien zu berücksichtigen.

Aufgabe der Erfindung ist es daher, Beleuchtungslichtleiter oder Bildleiter für Einweg-Endoskope bzw. Baugruppen mit Beleuchtungslichtleitern, Bildleitern und/ oder Kameras bereitzustellen, die insbesondere kostengünstig in der Herstellung sind und andererseits typische lichttechnische Anforderungen für Endoskope in der Medizintechnik, insbesondere eine hohe Transmission und eine hohe Farbwiedergabetreue ermöglichen. Dies bei gleichzeitig hoher Biokompatibilität und geringer Zytotoxizität entsprechend der medizintechnischen Anforderungen und Wirkungen.

Die Aufgabe der Erfindung wird dadurch gelöst, dass die proximalen und/ oder distalen Endflächen aus zumindest teil- oder abschnittsweise transparenten Kunststoff-Elementen bestehen oder ein transparenter Kunststoff an diese angeformt ist, wobei der transparente Kunststoff biokompatibel und/ oder nicht toxische Eigenschaften auf menschliche oder tierische Zellkulturen für Einwirkzeiten kleiner einem Tag aufweist. Damit können sehr kostengünstig Beleuchtungslichtleiter oder Bildleiter hergestellt werden, bei denen eine ansonsten aufwendige Endenbearbeitung, das heißt das Schleifen und Polieren der proximalen bzw. distalen Endflächen entfallen kann. Die Biokompatibilität bzw. die nicht toxischen Eigenschaften der Kunststoffe erlauben einen invasiven Eingriff im Körper (in vivo) oder ermöglichen In-Vitro-Untersuchungen an Zellkulturen oder Blutproben, ohne diese zu schädigen bzw. zu verändern. Durch die Wahl des Kunststoffes können hier optisch hochwertige Systeme bereitgestellt werden, die insb. den lichttechnischen Anforderungen für Endoskope gerecht werden, zumal die Temperaturbeständigkeit des Kunststoffes insbesondere für Einweg-Endoskope weniger hoch sein muß, was die Auswahl weniger einschränkt. Geeignete Kunststoffe sind Kunststoffe aus mindestens einer der Materialklassen Cyclo-Olefin-Co-Polymere, Polycarbonate, Polyethylen-Teraphthalaten, Perfluoralkoxy-Polymere, Polyvinylidenfluoride, Polymethylmethacrylate Polymethylmethacrylimide, Acryl- Styrol-Acrylnitril-Co-Polymere oder Raumtemperatur-vernetzende Silikon, heißvernetzende Flüssig-Silikone, Epoxid-Gießharze oder -Kleber, thermisch oder UV-vernetzende Acrylat-Gießharze, Polyurethan-Gießharze, Polyester-Gießharze oder aus Mischungen und/ oder Kombinationen derselben. Bei der Auswahl ist hier auf entsprechend biokompatible Varianten zu achten, die den eingangs erwähnten Norm-Anforderungen gerecht werden. Geeignet sind hier insbesondere thermoplastische Kunststoffe, die sich zum einen leicht spritzgießen lassen und transparent sind, beispielsweise PC, PMMA, COC etc., aber auch Kunststoffe, die als Gießharze applizierbar sind. Damit können entsprechend glatte Oberflächen mit sehr geringem Rauheitswert realisiert werden. Zudem sind o.g. Kunststoffe als biokompatible Version erhältlich.

Insbesondere zur mechanischen Anbindung an andere Komponenten des Endoskops kann vorgesehen sein, dass die proximale und/ oder die distale Endfläche zudem jeweils eine mechanische Schnittstelle in Form einer Hülsenkontur aufweist, welche aus Kunststoff besteht oder mittels Kunststoff-Spritzguß an den Beleuchtungslichtleiter oder Bildleiter angeformt ist, wobei dieser Kunststoff sich vom transparenten Kunststoff der proximalen oder distalen Endfläche zumindest teil- oder abschnittsweise hinsichtlich Material, Transparenz und/ oder Farbe unterscheiden kann. So lassen sich beispielsweise Kragen oder Absätze, aber auch Hinterschnittbereiche erzeugen, mit denen sich der Beleuchtungslichtleiter oder der Bildleiter mit einem Handstück und/ oder eines Schaftes des Endoskops verbinden lässt. Unter anderem können damit auch Rastverbindungen realisiert werden, die eine schnelle Montage ermöglichen, was wiederum die Herstellkosten senken kann.

Besonders bevorzugt sind Ausführungsvarianten, bei denen der transparente Kunststoff der proximalen und/ oder distalen Endflächen eine typischen Oberflächenrauigkeit Ra von ≤ 1,0 µm, vorzugsweise ≤ 0,5 µm, besonders bevorzugt ≤ 0,1 µm aufweist. Damit lassen sich Streuverluste an der Oberfläche minimieren, welche ansonsten zu einer Reduzierung der Beleuchtungsstärke bei Beleuchtungslichtleiter führen würden. Bei Bildleitern kann damit eine scharfe Abbildung des beleuchteten Objekts erreicht werden.

Weist der transparente Kunststoff der proximalen oder distalen Endflächen einen Brechungsindex auf, der im Wesentlichen dem des Kernmaterials der in dem verwendeten Beleuchtungslichtleiter oder Bildleiter verwendeten Fasern oder Faserkomponenten entspricht, können Reflektionsverluste minimiert werden, was bei den Beleuchtungslichtleitern zu einer Steigerung der Beleuchtungsstärke führt und bei Bildleitern Artefakte infolge von Reflektionen unterdrückt. Beträgt die Abweichung zwischen dem Brechungsindex der Fasern oder den Faserkomponenten und dem klar transparenten Kunststoff maximal ± 0,1, können bereits gute Ergebnisse erzielt werden. Bei einer Abweichung von maximal ± 0,05 beträgt sind die Brechungsindices bereits nahezu perfekt angepaßt, so dass die Reflektionsverluste bei den Beleuchtungslichtleitern vernachlässigt werden können. Bei den Bildleitern können insbesondere Geisterbilder infolge von Mehrfachreflektionen ausgeschlossen werden.

In vorteilhafter Ausgestaltung der Erfindung umfassen die Beleuchtungslichtleiter und/ oder Bildleiter für Endoskope Faserbündel aus Glasfasern, Quarzfasern oder Kunststoff-Fasern. Glasfasern sind insbesondere zur Übertragung von Licht oder Bildinformationen im sichtbaren Spektralbereich bis in den nahen IR-Bereich geeignet. Dieses trifft auch für Kunststofffasern zu, wobei allerdings die Anwendungslänge der Kunststofffasern auf typischerweise wenige Zentimeter bis etwa max. 1 m begrenzt sind. Quarzfasern kommen insbesondere dann zum Einsatz, wenn die Anwendungswellenlänge in den IR-Bereich bis typischerweise 2,2 µm hineinreicht oder auch Lichtanteile im nahen UV-Bereich, unterhalb etwa 400 nm genutzt werden sollen. Dies ist insbesondere bei Fluoreszenz-Anwendungen von Interesse. Besonders vorteilhaft ist es dabei, wenn die Bündel oder Einzelfasern zumindest teil- oder abschnittsweise mit einem Mantel, Schlauch, Schrumpfschlauch oder Netzschlauchgewebe umgeben sind oder mittels eines Schaftes des Endoskops geschützt ist. Dies erhöht die mechanische Robustheit des Systems.

Dabei kann vorgesehen sein, dass der Mantel aus einem weiteren Kunststoffmaterial besteht und als extrudiertes Kabel ausgeführt ist. Derartige Kabel lassen sich besonders kostengünstig in einem Endlos-Prozess herstellen.

Insbesondere können sowohl für das Kabel als auch für die Hülsen in den oben genannten Ausführungsvarianten kostengünstige, weniger temperaturstabile Kunststoffe eingesetzt werden, da bei den Single-Use-Anwendungen keinerlei insbesondere thermische/ chemische Aufbereitungsprozesse, wie Autoklavieren (typ. 130 bis 140°C in gesättigtem Wasserdampf) und/ oder Thermodesinfektor-Prozessen (bis 95°C, Reinigungsmittel mit pH 11) erforderlich sind. I.d.R. werden zur Sterilisation von Einweg-Instrumenten Ethylenoxid-Begasungen oder zum Teil Plasma-basierte Gas-Sterilisationen (STERAD, mit Wasserstoffperoxid und Plasma, oder STERIS, nur mit Wasserstoffperoxid) eingesetzt, welche bei max. 60° C stattfinden.

Der Kunststoff für den extrudierten Mantel kann aus einem zumindest teil- oder abschnittsweise transluzenten, opaken oder eingefärbten Kunststoff bestehen. Damit kann beispielsweise mit einer seitlich abstrahlenden Lichtleitfaser eine seitliche Beleuchtung am Endoskop ermöglicht werden.

Ist der Beleuchtungslichtleiter oder Bildleiter aus flexiblen oder semi-flexiblen Faserbündeln ausgeführt und der Mantel zumindest teil- oder abschnittsweise als starre Hülle ausgeführt, kann damit ein Schaft für ein starres Endoskop realisiert werden.

Die Erfindung bezieht sich auch auf starre faseroptische Licht- oder Bildleiter, zum Beispiel in Form von gezogenen Faserstäben oder in Form von gepreßten Faserstäben, welche in vorteilhafter Weise ebenfalls auf den gleichen Glassystemen basiert, wie sie für entsprechende flexible Glasfaserbündel verwendet werden. Auch hier können mit einer direkt angeformten Kunststoffkappe am proximalen und/ oder distalen Ende des Lichtleiters optische Elemente und / oder Hülsen kostengünstig ausgebildet werden.

Besonders vorteilhaft ist es, wenn die Glasfasern, Faserstäbe oder gepreßten Faserstäbe aus einem Pb- bzw. Schwermetall-freien Kernglas und Mantelglas bestehen. Derartige Fasersysteme bieten insbesondere eine hohe Transmission im VIS-Spektralbereich und zeigen aufgrund der vergleichsweisen hohen Transmission im blauen Spektralbereich eine hohe Farbtreue, was insbesondere wichtig ist bei der medizinischen Beurteilung von Gewebe. Oft entscheiden hier nur geringfügige Farbunterschiede des Gewebes, ob es sich um eine gutartige oder bösartige Gewebeveränderung handelt. Daher kommt es auf einen hohen CRI-Wert des Gesamtsystems aus Lichtquelle, Beleuchtungslichtleiter und bildgebender Einrichtung an, wobei CRI (Color Rendering Index) eine Kennzahl einer photometrischen Größe ist, mit der die Qualität der Farbwiedergabe von Lichtquellen gleicher korrelierter Farbtemperatur beschrieben wird. Ein CRI-Wert von > 90 kann mit den oben beschriebenen Glasfasern, Faserstäbe oder gepreßten Faserstäbe erzielt werden. Derartige Fasersysteme sind seitens der Anmelderin unter dem Namen SCHOTT PURAVIS^{®} bekannt und sind hinsichtlich ihrer Zusammensetzungen in der DE 102012100233 B4 und DE 102013208838 B4 beschrieben. Ähnliche Fasersysteme sind auch in der EP 2072477 B1 beschrieben, welche ebenfalls Pb-frei sind.

Insbesondere für die Verwendung in Endoskopen ist es von Vorteil, wenn Glasfasern, Faserstäbe oder gepreßten Faserstäbe aus einem Glassystem bestehen, welches für das zu leitende Licht einen Akzeptanzwinkel 2α von größer 80°, besonders bevorzugt von größer 100° aufweist. Zum einen kann erreicht werden, dass insbesondere Licht von LEDs, welche i.d.R. einen sehr breiten Abstrahlwinkel aufweisen, ohne komplexe Optiken am proximalen Ende in die Glasfasern bzw. Faserstäbe oder gepreßten Faserstäbe einkoppelbar sind, ohne dass erhöhte Einkoppelverluste entstehen. Andererseits kann am distalen eine weitwinklige Ausleuchtung ohne zusätzlich erforderliche Optik erreicht werden, was insbesondere endoskopische Untersuchungen bevorzugt. Damit kann eine optimale Ausleuchtung bei den derzeit üblichen Kamera-Sichtwinkeln (üblicherweise 120° diagonal) erzielt werden.

In einer besonders bevorzugten Ausführungsvariante ist vorgesehen, dass die distale und/ oder proximale Endfläche mit der mechanischen Schnittstelle als Hülse ausgeführt ist, welche separat hergestellt ist, und mittels eines Klebers auf das Faserbündelende oder Faserstabende des Beleuchtungslichtleiters oder des Bildleiters fixiert ist, wobei der Kleber als wärmeaushärtender oder UV-Licht-aushärtender Kleber ausgeführt ist, welcher einen optischen Brechungsindex aufweist, der im Wesentlichen dem des Kernmaterials der in den verwendeten Beleuchtungslichtleiter oder Bildleiter verwendeten Fasern oder Faserkomponenten entspricht und die Abweichung dazu maximal ± 0,1, bevorzugt maximal ± 0,05 beträgt und wobei der Brechungsindex der Hülse geringfügig geringer ist als der des Klebers. Hiermit können hohe Koppeleffizienzen erzielt werden. Ein geringfügig niedrigerer Brechungsindex der Hülse gegenüber dem des Klebers hilft, Abstrahlverluste seitlich aus der Hülse zu minimieren. Derartige Hülsen lassen sich kostengünstig als Spritzgußteil, hier insbesondere als Präzisionsspritzgußteil herstellen. Die komplette Funktionalität hinsichtlich der Aufnahme der Fasern, der mechanischen Schnittstelle und der Ausbildung der proximalen bzw. distalen Endfläche hinsichtlich ihrer Topographie kann dabei im Spritzguss-Werkzeug implementiert werden. Werden wärmeaushärtende oder UV-Licht-aushärtende Kleber verwendet, können kurze Prozesszeiten im Bereich von typisch kleiner 60 s beim Montieren bzw. Verkleben der Faserkomponenten realisiert werden, was zudem die Herstellkosten senken kann.

Dabei ist in einer besonders vorteilhaften Ausführungsform vorgesehen, dass die Hülse Aufnahmeabschnitte zur Aufnahme von Faserbündeln aufweisen, die von einem zunächst leicht konischen Abschnitt in einen Abschnitt münden, der im Wesentlichen parallel angeordnete Seitenwände aufweist und die Hülse weiterhin Aufnahmen für elektronischen Komponenten aufweist, und diese Aufnahmeabschnitte den Bereich der Aufnahme für elektronische Komponenten zumindest bereichsweise umschließen. Damit können beispielsweise Anordnungen von Fasern und elektronischen Komponenten realisiert werden, bei denen ein elektronisches Bauteil von einer proximalen oder distalen Endfläche von dieser umschlossen ist. Weiterhin sind auch im Wesentlichen U-förmige Anordnungen oder Anordnungen denkbar, oder Anordnungen, bei denen das elektronische Bauteil von zwei D-förmig ausgebildeten distalen oder proximalen Endflächen gegenüberliegend umrandet ist.

Darüber hinaus sind auch 3- oder 4-geteilte distale oder proximale Endflächen denkbar, die das elektronische Bauteil als kreisförmige oder ovale beziehungsweise nierenförmige Austrittsflächen umschließen. Die komplette Funktionalität der Faserfixierung und Ausrichtung sowie der Anordnung der Endflächen lassen sich dabei in das technische design der Hülse legen beziehungsweise im Werkzeugdesign implementieren. Aufgrund der sehr geringen Abmessungen sind hierbei insbesondere Präzisionsspritzgieß-Werkzeuge beziehungsweise Maschinen von Vorteil.

Eine alternative Ausführungsvariante sieht vor, dass die distale und/ oder proximale Endfläche mit der mechanischen Schnittstelle in Form einer Hülse mittels Spritzguss auf zuvor abgelängte Kabelabschnitte angeformt ist, wobei dieser Prozess als zweistufiger Prozess ausgelegt sein kann, wobei in einem ersten Schritt das Kabelende zumindest an zwei gegenüberliegenden Stellen mittels der Außenkontur des Kabels angepaßten Werkzeugen fixiert und zumindest teil- oder abschnittsweise mit einem ersten Kunststoffes umspritzt ist, und in einem zweiten Schritt die Hülsengeometrie mittels eines zweiten Kunststoffes angeformt ist, wobei in einem der Schritte die distale und/ oder proximale Endfläche mit dem klar transparenten Kunststoff ausformbar ist. Ein zweistufiger Prozess kann verhindern, dass die Fasern beim Spritzgießprozess, der üblicherweise mit Drücken von vielen 10 bar einhergeht, unkontrolliert aufspleißt. Mit dem ersten Prozessschritt kann zumindest eine Art fester Kragen am Ende des Kabelabschnitts um das Kabel erzeugt werden, der das Aufspleißen verhindert. Als Kunststoffe hierfür können auch opake beziehungsweise eingefärbte Kunststoffe verwendet werden. Im zweiten Schritt wird dann mit dem klar transparenten Kunststoff die eigentlichen proximalen und/ oder distalen Endflächen erzeugt.

Ein besonders kostengünstiger Prozess, welcher insbesondere für hohe Stückzahlen von Vorteil ist, wenn in einem Endlos-Prozess auf ein zuvor extrudiertes Kabel in bestimmten Abständen entsprechend der finalen Bauteillänge eine hinsichtlich ihrer Kontur doppelten Hülse als mechanische Schnittstelle angeformt ist, welche dann in einem nächsten Prozessschritt trennbar ist, in die damit erzeugten Kabelabschnitte mittels ein oder mehreren weiteren Spritzgieß-Prozessen mit klar transparenten Kunststoff die proximalen und/ oder distalen Endflächen anformbar sind. Hiermit kann eine nahezu vollautomatisierte Fertigung realisiert werden, was insbesondere eine sehr kostengünstige Bereitstellung derartiger Lichtleiter ermöglicht.

Eine weitere alternative Ausführungsform sieht vor, dass ein zuvor extrudiertes Kabel in bestimmten Abständen entsprechend der finalen Bauteillänge geteilt ist oder ein entsprechender Faserbündelabschnitt, welcher mit einem Schlauch oder Schrumpfschlauch umgeben ist, und die im inneren des extrudierten Kabelabschnitts oder Faserbündelabschnitts vorhandenen Faserbündel nach innen verschoben sind und der Raum zwischen Faserbündelende und Mantelrand oder Rand des Schlauchs oder Schrumpfschlauches mit einem klar transparenten selbst nivellierendem Kunststoff gefüllt ist. Insbesondere mit Gießharzen lassen sich damit Lichteintritts- bzw. Lichtaustrittsflächen realisieren, die eine hinreichend glatte Oberfläche ausbilden.

Alternativ kann vorgesehen sein, dass ein zuvor extrudiertes Kabel in bestimmten Abständen entsprechend der finalen Bauteillänge geteilt ist oder ein entsprechender Faserbündelabschnitt, welcher mit einem Schlauch oder Schrumpfschlauch umgeben ist, und der Kabelmantel, der Schlauch oder Schrumpfschlauch gegenüber dem Faserbündel gelängt und die dabei entstehende Kavität mit optisch klarem Kunststoff aufgefüllt ist oder ein vorgefertigtes klar transparentes Kunststoffteil oder ein Lichtleitstab oder Faserstab aus Glas oder Kunststoff in die Kavität eingesetzt und fixiert ist. Auch damit lassen sich entsprechende Lichteintritts- beziehungsweise Lichtaustrittsflächen realisieren.

In einer Ausführungsvariante kann auch vorgesehen sein, dass der Mantelabschnitt, Schlauch- oder Schrumpfschlauchabschnitt, der die Kavität ausbildet, verformt ist und eine bestimmte Lichteintritts- oder Lichtaustrittskontur nach Aushärten des Kunststoffes oder nach Einsetzen des Kunststoffteils oder des Lichtleitstabs ausbildet. Dies kann mittels spezieller Werkzeuge geschehen. Damit können unterschiedliche proximale bzw. distale Konturen erzeugen, welche beispielsweise zur Aufnahme eines Kamera-Chips oder eines Arbeitskanals am distalen Ende genutzt werden können.

Besonders vorteilhaft hinsichtlich einer kostengünstigen aber auch platzsparenden Lichtleiter-Ausführung ist es, wenn aktive elektronische Elemente in Form von LEDs, Sensoren oder Kamera-Chips in die angeformten Hülsen integrierbar oder an diese mittels einer Rastverbindung ansteckbar sind. So lassen sich LED-Elemente beispielsweise in der proximalen Endhülse integrieren und ermöglichen so eine besonders hohe Einkoppel-Effizienz, was sich besonders vorteilhaft hinsichtlich der Beleuchtungsstärke am distalen Ende des Lichtleiters bemerkbar macht. Als LEDs können neben Weißlicht-LEDs auch RGBW-LEDs zum Einsatz kommen, bei denen zwischen verschiedenen Farben umgeschaltete werden kann. Dies ermöglicht neben einer normalen Betrachtung von Gewebe auch bestimmte diagnostische Untersuchungen, bei denen das Gewebe mit bestimmten Wellenlängen untersucht wird. Denkbar ist auch die Kombination von Weißlicht- beziehungsweise RGBW-LEDs mit LEDs, die im tief blauen Spektralbereich (zum Beispiel 405 nm) oder im nahen UV-Bereich emittieren. Damit können auch Fluoreszenz-Anregungen ermöglicht werden. Hinsichtlich eines Wärmemanagements kann vorgesehen sein, dass die LEDs mittels Metallstifte mit Wärmesenken im Handstück des Endoskops thermisch verbunden sind. Die Integration eines Kamera-Chips in der distalen Endhülse (Chip on Tip) ermöglicht, dass die zu untersuchende Gewebeoberfläche direkt abgebildet werden kann.

Vorteilhaft kann es sein, wenn die proximalen und/ oder distalen Endflächen als optisches Element zur Erzielung einer bestimmten Strahlformung ausgeführt sind, und dabei eine plane, konvexe, konkave Fläche oder eine hinsichtlich ihrer Topographie beliebig gestaltete Freiformfläche aufweisen. Durch eine entsprechende Werkzeuggestaltung kann beispielsweise zur besseren Lichteinkopplung die proximale Hülse mit Kondensor-Linsen ausgestattet sein, um beispielsweise das Licht der in der Regel eher breit abstrahlenden LEDs zu bündeln und entsprechend der numerischen Apertur der Fasern (zwischen 0,55 und 0,70; zum Beispiel SCHOTT PURAVIS^{®} GOF70 mit einer numerischen Apertur von 0,57, SCHOTT PURAVIS^{®} GOF85 mit einer numerischen Apertur von 0,68) in diese einzukoppeln. Eine entsprechende Ausbildung einer konvexen Linse am distalen Ende kann auch vorteilhaft genutzt werden, beispielsweise eine abbildende Optik für den Kamera-Chip zu realisieren. Weiterhin kann auch eine Weitwinkel-Abstrahlcharakteristik, zum Beispiel mit sphärischer oder ringförmiger Abstrahlcharakteristik, am distalen Ende des Lichtleiters mit derart ausgebildeten optischen Elementen ermöglicht werden. Mit einer sphärischen Abstrahlcharakteristik kann beispielsweise eine homogene Ausleuchtung von Körperhohlräumen erfolgen.

Eine bevorzugte Ausführungsvariante sieht vor, dass zusätzliche Elemente aus Glas oder Kunststoff zur Abdeckung der aktiven elektronischen Elemente an den proximalen oder distalen Endflächen vorgesehen sind. Damit kann eine zusätzliche elektrische Isolation und/oder Abschirmung erzielt werden, mit der insbesondere Applikationen mit erhöhten Isolations- bzw. Ableitstromanforderungen adressiert werden können.

Zudem kann vorgesehen sein, dass die distale Hülse mit dem Kamera-Chip als 2-Komponenten-Spritzguß-Teil ausgebildet ist, wobei die den Kamera-Chip aufnehmenden Abschnitt als schwarz eingefärbtes bzw. opakes Kunststoffmaterial ausgebildet ist und die distale Endfläche aus transparentem Kunststoff besteht. Damit kann eine zusätzliche Abschirmung des Kamera-Chips hinsichtlich Streulicht erzielt werden.

Im Zusammenhang mit Einweg-Endoskopen für die Medizintechnik kann es besonders vorteilhaft sein, wenn sogenannte Hybrid-Kabel zum Einsatz kommen, bei denen neben optischen Licht- und/ oder Bildleitelementen auch elektrische Leiter in einem Kabel geführt sind. Damit können beispielsweise Kamera-Chips mit Spannung versorgt bzw. Bildinformationen zu einer Auswerteeinheit übertragen werden.

In einer Ausführungsvariante kann vorgesehen sein, dass das extrudierte Kabel für den Beleuchtungslichtleiter oder Bildleiter als Multilumen-Kabel ausgeführt ist, welches unterschiedliche Kammern aufweist, mit dem ein Faserbündel, einzelne Quarz-Fasern, Medien in Form von Gasen oder Flüssigkeiten in einem Fluid-Kanal und/ oder elektrische Leitungen getrennt führbar sind. Besonders vorteilhaft ist hier die trennbare, voneinander unabhängige Integration von Licht- beziehungsweise Energieführenden Komponenten, die so auf kleinstem Raum eine hohe Funktionalität ermöglichen. So können die Faserbündel zur Lichtführung, Quarzfasern Beispielsweise zur Energieübertragung eines Laserstrahls genutzt werden. Die elektrischen Leitungen können dabei zur Weiterleitung von Bildsignalen des Kamera-Chips an einen Monitor genutzt werden. Derartige Multilumen-Kabel lassen sich mittels entsprechender Extrudier-Werkzeuge sehr kostengünstig herstellen.

Dabei kann vorgesehen sein, dass das Multilumen-Kabel einen flexiblen Abschnitt des Endoskops oder das Multilumen-Kabel aus einem bei Raumtemperatur starren Kunststoff besteht und somit einen starren Schaft des Endoskops ausbildet. Damit lassen sich besonders kostengünstig flexible oder starre Einweg-Endoskope realisieren.

Ist das Multilumen-Kabel in einem Koextrusionsprozess gezielt segmentweise transparent oder opak ausgeführt, können beispielsweise auch Beleuchtungs- oder optische Detektionsaufgaben erfüllt werden. Das Multilumenkabel kann dabei zumindest teil- oder abschnittsweise, auch innerhalb einzelner Lumen, aus elektrisch leitfähigen Materialien, bspw. entsprechend gefüllten Kunststoffen ausgeführt sein und/oder von elektrisch leitfähigen Materialien umgeben sein.

All die zuvor ausgeführten Beispiele sind geeignet, entsprechend kostengünstige faseroptische Bauteile beziehungsweise Baugruppen bereitzustellen, welche in flexiblen oder starren Einweg-Endoskope verbaut werden können. Der Sammelbegriff Einweg-Endoskope umfaßt hier alle medizintechnische Geräte mit denen einerseits Licht in das Innere des Körpers geleitet wird und anderseits mittels Optiken, Bildleitern oder Kamera-Chips eine Bildinformation an den Operateur ausgegeben wird. Dies können beispielhaft Angioskope für Gefäßuntersuchungen mit flexiblem Endoskop, Laparoskope für Untersuchungen in der Bauchhöhle und Arthroskope für Gelenkuntersuchungen mit je starrem Endoskop, sowie Ohr-Endoskop, Rhino-Endoskop, Sinuskop oder Osopharyngoskop für HNO-Untersuchungen mit jeweils starrem Endoskop sein.

Hier können die zuvor beschriebenen Ausführungsvarianten der Beleuchtungslichtleiter und/ oder Bildleiter in einem Handstück des Endoskops integriert werden und können, teilweise direkt einen flexiblen Abschnitt oder einen Schaft des Endoskops ausbilden, je nach Bauart des Endoskops. Durch den Wegfall der teils sehr aufwendigen Schleif- und Polierprozesse und durch die erleichterte Montage selbst können damit Kosten eingespart werden.

Eine weitere Verwendung insbesondere der Beleuchtungslichtleiter, wie sie zuvor in den verschiedenen Ausführungsvarianten beschrieben wurden, sieht neben dem Einsatz im Medizingeräte-Bereich auch den Einsatz für In-vitro-Diagnostik-Geräte vor. Dabei können derartige Lichtleiter auch als Detektorlichtleiter eingesetzt werden. Hier werden beispielsweise oft eine große Zahl derartiger Beleuchtungsbeziehungsweise Detektorlichtleiter in einem Gerät zum Beispiel zur parallelen Untersuchungen an Blutproben verwendet. Hier sind insbesondere die Kostenvorteile, sei es infolge einer Reduzierung des Montageaufwandes oder der Integration von Zusatzfunktionen, zu nennen. Eine biokompatible Ausführung der Kunststoffe kann hier direkt genutzt werden um beispielsweise Blutproben oder Zellkulturen unmittelbar in Kontakt mit den Beleuchtungs- bzw. Detektorlichtleitern zu bringen. Zudem werden mit den zuvor beschriebenen Glas- oder Quarzfasern aufgrund Ihrer Vorteile bei der optischen Übertragung spektroskopische Untersuchungen und/ oder auch Untersuchungen mittels Fluoreszenzanregung ermöglicht.

Als weitere Verwendungsbeispiele sind hier unter anderem zu nennen: Beleuchtungslichtleiter in Hausgeräten (Herde, Spülmaschinen, Kühl-/Gefrier-Schränke, Backöfen etc.) beziehungsweise Küchen-Klein-Geräte (Mixer, Toaster, Auftisch-Kochgeräten, Kaffee-Automaten etc.) zum Beispiel zur Kenntlichmachung von Betriebszuständen und/ oder zur Beleuchtung von Garräumen oder Innenräumen, insbesondere dann wenn diese mit Lebensmitteln in Kontakt kommen; Home-Ambiente-Beleuchtung; Automotive Exterieur-/Interieur-Beleuchtung.

Die Erfindung wird im Folgenden anhand eines in den Figuren dargestellten Ausführungsbeispiels näher erläutert. Es zeigen:
Figur 1 schematisch stark vereinfacht ein Einweg-Endoskop, welches als flexibles Endoskop ausgeführt ist,
Figur 2 schematisch stark vereinfacht ein Einweg-Endoskop, welches als starres Endoskop ausgeführt ist,
Figur 3 schematisch einen Beleuchtungslichtleiter mit aufgeklebter distaler Hülse,
Figur 4 schematisch einen Beleuchtungslichtleiter mit angeformter distaler Hülse,
Figur 5 schematisch einen Beleuchtungslichtleiter mit distaler Hülse und integriertem Kamera-Chip,
Figur 6a bis 6c schematisch verschiedene Anordnungen einer distalen Endfläche mit einem Kamera-Chip,
Figur 7 in einer schematischen Schnittdarstellung eine distale Hülse mit einer Anordnung gem. Figur 6a,
Figur 8 schematisch einen Beleuchtungslichtleiter mit proximaler Hülse und darin integrierter Beleuchtungseinrichtung,
Figur 9 in einer stark vereinfachten Prozess-Sequenz eine Herstellmethode eines Beleuchtungslichtleiters und
Figur 10 schematisch ein Multilumen-Kabel zur Aufnahme unterschiedlicher Komponenten bzw. Funktionalitäten.

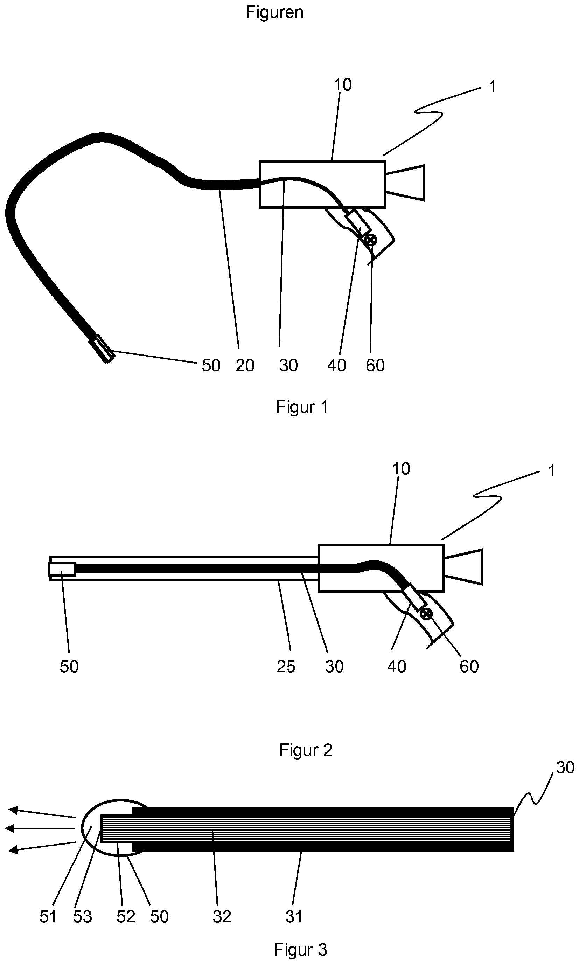

Figur 1 zeigt schematisch den Aufbau eines Endoskope 1 gemäß der Erfindung. Beispielhaft ist hier ein einfaches flexibles Endoskop 1 stark vereinfacht dargestellt, welches ein Handstück 10 und einen flexiblen Abschnitt 20 aufweist, wobei der flexible Abschnitt 20 zum Beispiel in einen Körper-Hohlraum einführbar ist. Schematisch dargestellt ist hier ein Beleuchtungslichtleiter 30, der eine proximale Hülse 40 an einer als LED 60 ausgeführten Beleuchtungseinrichtung im Handstück 10 und eine distale Hülse 50 am Ende des flexiblen Abschnitts 20 aufweist. Das Licht der LED 60 wird an der Endfläche der proximalen Hülse 40 eingekoppelt und über den Beleuchtungslichtleiter 30 zur distalen Hülse 50 geführt, und kann dann über entsprechende Auskoppeloptiken in das Körperinnere abgestrahlt werden. Nicht dargestellt sind in Figur 1 die bildgebenden Komponenten. Diese können beispielsweise C-MOS-Kameras sein, die in die distale Hülse 50 integriert sind, und die Bildinformation zu einem ebenfalls nicht dargestellten Monitor elektrisch übertragen. Ebenso denkbar sind faseroptische Bildleiter, die die Bildinformation zu einer Kamera oder direkt zu einer Okularoptik übertragen. Derartige Bildleiter bestehen aus mehreren Tausend feiner, nur wenige µm dicke Einzel-Glasfasern, die entsprechend pixelweise die Bildinformation übertragen.

Je nach Endoskop-Typ und Anwendung sind folgende typischen Abmessungen für derartige Lichtleiter denkbar: Länge zwischen 100 mm und 3000 mm, typ. 500 bis 1000 mm, Lichtleiter-Durchmesser zwischen 0,5 mm und 5 mm, typ. zwischen 1 und 2 mm.

Figur 2 zeigt ebenfalls schematisch und stark vereinfacht ein Endoskop 1, welches als starres Endoskop 1 ausgeführt ist. Der Beleuchtungslichtleiter 30 ist hier in einem starren Schaft 25 geführt. Auch hier sind die bildgebenden bzw. bildübertragenden Komponenten, wie sie zuvor beschrieben wurden, der Übersicht wegen nicht dargestellt.

Im Folgenden werden insbesondere Ausführungsbeispiele beziehungsweise Herstellverfahren beschrieben, die sich auf Beleuchtungslichtleiter 30 beziehen. Grundsätzlich können diese auch auf die Bildleiter übertragen werden.

In Figur 3 ist ein Beleuchtungslichtleiter 30 in einer Teilansicht mit einer distalen Hülse 50 dargestellt. Der Beleuchtungslichtleiter 30 besteht in diesem Fall aus einem extrudierten Kabel 31, wobei ein Mantel aus Kunststoff ein Faserbündel 32 umschließt.

Die Faserbündel-Terminierung erfolgt hier derart, dass der Mantel des extrudierten Kabels 31 am Ende ab isoliert wird, und eine zuvor in einem Spritzgießprozess hergestellte klar transparente Hülse als distale Hülse 50 mit seinem Aufnahmeabschnitt 52 auf das freiliegende Faserbündel 32 geschoben wird und mit zuvor in diese Hülse appliziertem klar transparenten Harz zum Beispiel in Form eines vorzugsweise schnell heißvernetzenden oder UV-vernetzenden Klebers die Hülse fixiert wird. Damit ist die distale Endfläche 53 des Faserbündels 32 mit einem klar transparenten Kunststoff abgedeckt. Diese Art der Terminierung lässt sich auch auf die proximale Hülse 40 des Beleuchtungslichtleiters 30 übertragen. In diesem Fall kann damit die proximale Endfläche 43 mit einem klar transparenten Kunststoff abgedeckt werden.

Zudem können die proximalen und distalen Hülsen 40, 50 mechanische Schnittstelle 44, 54 aufweisen, die aus der äußeren Kontur der proximalen und distalen Hülsen 40, 50 heraus resultieren. Dies können umlaufende Nuten, Rastnasen, Einkerbungen, Flansche und dergleichen sein.

Diese Hülsen können neben einer planen Endfläche auch als optische Elemente 51 in Form von Linsen (konvex oder konkav) oder als irregulär ausgebildete Endfläche zur Strahlformung ausgeführt sein. Figur 3 zeigt lediglich schematisch die distale Hülse 50 mit einem optischen Element 51 in Form einer beim Spritzgießprozess ausgeprägten Linsenkuppe, mit der das austretende Licht zum Beispiel gebündelt werden kann. Die Funktionalität der Ein- und/ oder der Auskoppelhülse bzw. der proximalen und/ oder distalen Hülse 40, 50 kann besonders kostengünstig in das Design des Werkzeuges implementiert werden und ermöglicht eine extrem kostengünstige Terminierung der proximalen bzw. distalen Endflächen 43, 53.

Das Faserbündel 32 des Beleuchtungslichtleiters 30 oder auch des Bildleiters kann aus Glasfasern (GOF), Quarzfasern oder Kunststoff-Fasern (POF) bestehen, welches mit einem extrudierten Mantel, wie in Figur 3 dargestellt, einem Schlauch oder Netzschlauchgewebe umgeben ist. Der Mantel-Kunststoff des extrudierten Kabels 31 besteht aus einem opak eingefärbten Kunststoff. In einer weiteren Ausführungsform kann das Faserbündel 32 selbst und/oder dessen einzelne Fasern zumindest teil- oder abschnittsweise eine elektrisch leitfähige Beschichtung aufweisen und/oder der Mantel-Kunststoff zumindest teil- oder abschnittsweise aus oder mit einem elektrisch leitfähigen Material ausgebildet sein.

Die nachfolgende Tabelle zeigt in einer Materialübersicht Kunststoffe, welche für den Mantel des Kabels 31 sowie für die klar transparente Abdeckung der proximalen bzw. distalen Endfläche 43, 53 beziehungsweise für die proximale bzw. distale Hülse 40, 50 geeignet sind.

Bei den thermoplastischen Elastomeren (TPE) unterscheidet man folgende Gruppen:
- TPE-A oder TPA = Thermoplastische Co-Polyamide
- TPE-E oder TPC = Thermoplastische Polyesterelastomere / Thermoplastische Co-Polyester
- TPE-O oder TPO = Thermoplastische Elastomere auf Olefin-Basis, vorwiegend PP/EPDM
- TPE-S oder TPS = Styrol-Blockcopolymere (SBS, SEBS, SEPS, SEEPS und MBS)
- TPE-U oder TPU = Thermoplastische Elastomere auf Urethanbasis
- TPE-V oder TPV = Thermoplastische Vulkanisate oder vernetzte thermoplastische Elastomere auf Olefin-Basis, vorwiegend PP/EPDM.

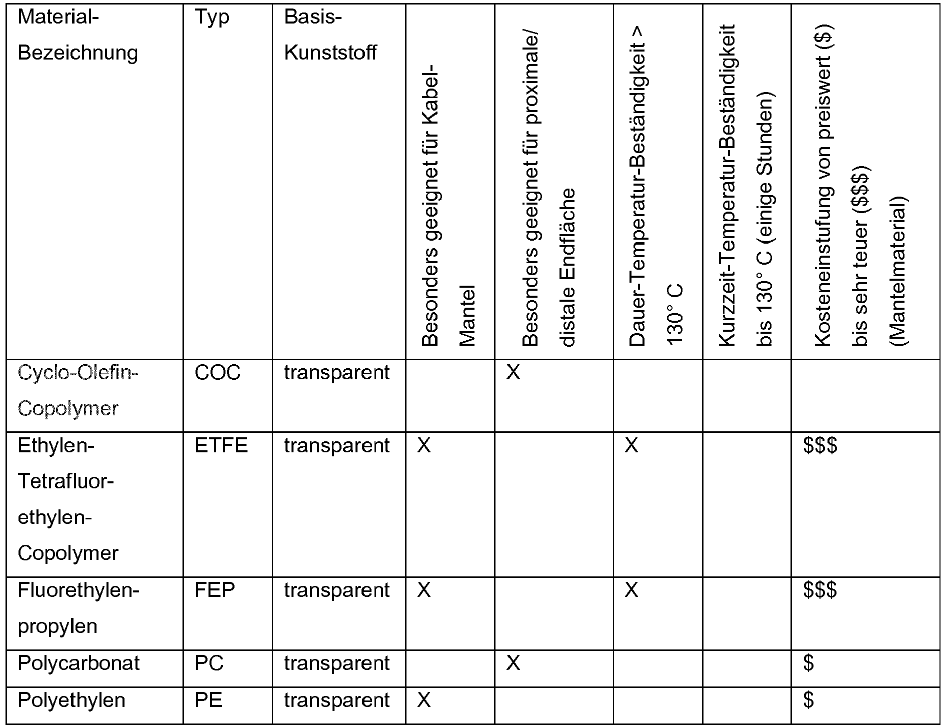

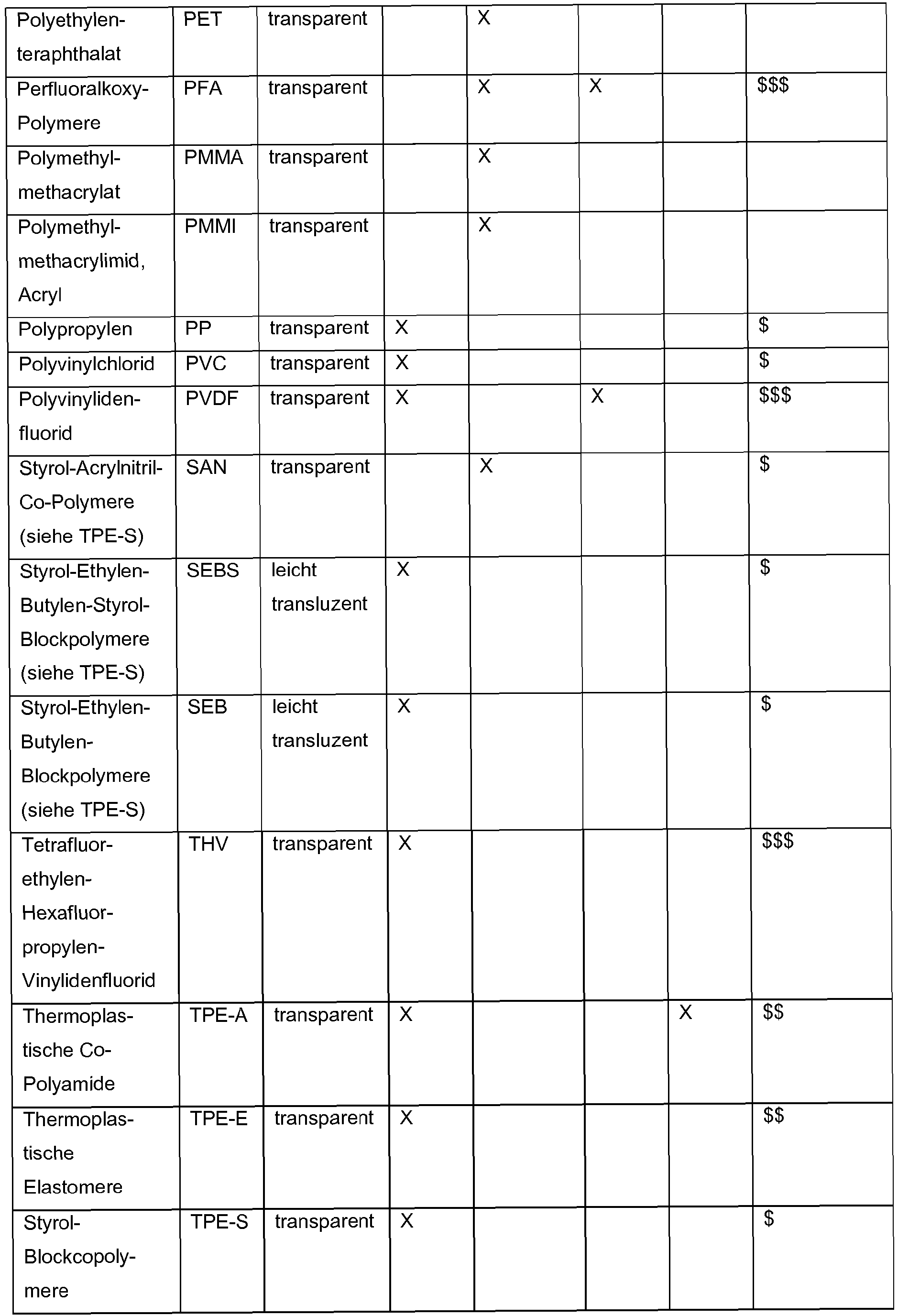

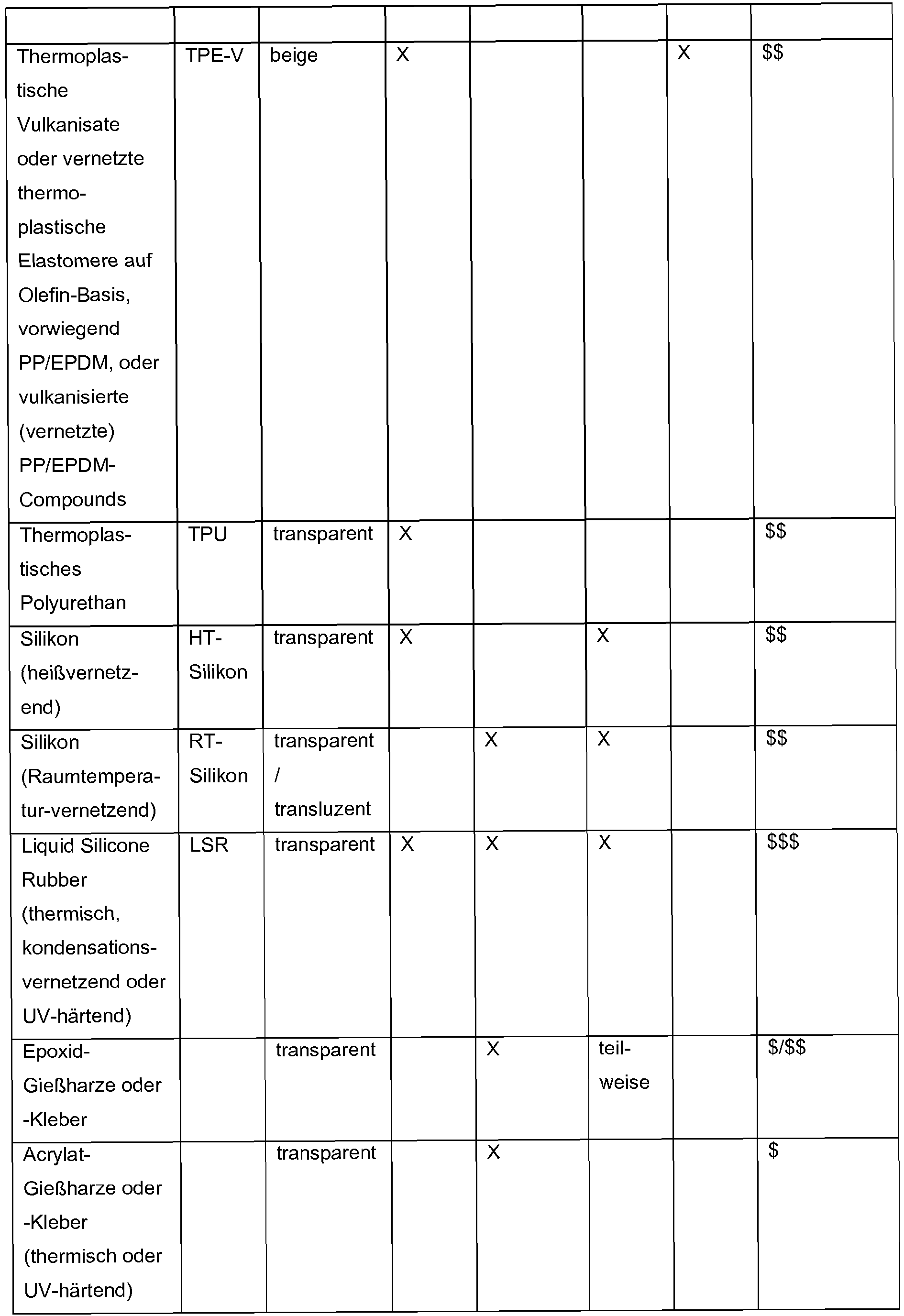

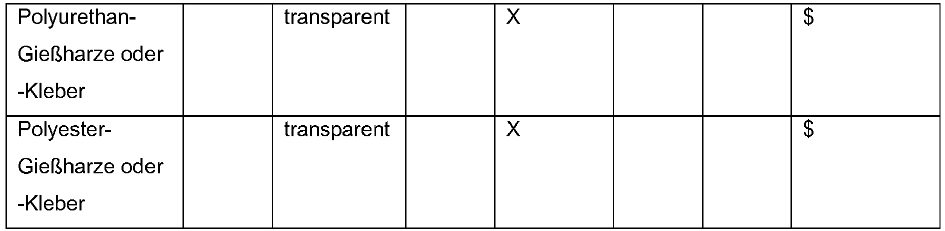

Insbesondere die Kunststofftypen TPE-E, TPE-V und TPE-U sind für eine Extrusion besonders interessant, da diese eine sehr gute Extrudierbarkeit aufweisen und insbesondere eine gute bis sehr gute Eignung für einen medizinischen Einsatz besitzen. Hinsichtlich einer kostengünstigen Herstellung besitzen diese Materialien zudem noch vergleichsweise günstige Materialkosten. Günstige Kunststoffe wie PVC, Compounds und Blends aus PP, PE, TPE-S (SEBS) haben teilweise gerade im Bereich Temperaturbeständigkeit zwar erhebliche Defizite. Meist sind diese nicht über 100° C einsetzbar. Allerdings liegen die Temperaturanforderungen für Einweg-Endoskope deutlich darunter, so dass diese Materialien auch aufgrund ihrer niedrigen Materialkosten und problemlosen Verarbeitung für diesen Einsatz besonders geeignet sind. Die sonst übliche Mindest-Temperaturbeständigkeit von größer 133° C bis 137° C, was der Temperaturbandbreite beim Autoklavieren für wiederverwendbaren bzw. aufbereitbaren medizinischen Geräten beziehungsweise Komponenten entspricht, ist hier nicht erforderlich, da für die bei Einweg-Medizinprodukten üblichen Sterilisationsprozesse, i.d.R. Prozesse verwendeten werden, welche nur im Bereich von Raumtemperatur bis max. 60° C ablaufen. Als Beispiel einer üblicherweise eingesetzten Sterilisationsmethode ist hier die Begasung mit Ethylen-Oxid zu nennen.

Die Gruppe der preiswerten und mittelteuren Kunststoffe stehen in der Regel meist in einer großen Bandbreite hinsichtlich ihrer Elastizität und Härtegrades zur Verfügung beziehungsweise können durch Mischen von mehreren Kunststofftypen zu einem Poly-Blend gewünschter Performance hergestellt werden. Dies hat den Vorteil gegenüber den "teuren" Kunststoffen, beispielsweise FEP, PVDF, dass sich hieraus Beleuchtungslichtleiter 30 mit fast identischen Eigenschaften jedoch unterschiedlicher Flexibilität herstellen lassen.

Die teuren Kunststoffe, zum Beispiel FEP, PFA, PVDF, sind zwar universell einsetzbar und besitzen insbesondere eine hohe Dauertemperaturbeständigkeit, oft kombiniert mit einer hohen chemischen Beständigkeit, sind jedoch nur sehr eingeschränkt mit anderen Kunststoffen zu kombinieren oder als Poly-Blend zu mischen, um zum Beispiel die Flexibilität zu erhöhen.

Alle genannten Kunststoffe werden mehr oder weniger bereits für medizinische Produkte eingesetzt.

Als Material für die transparenten Hülsen ist neben PC und PA auch COC sehr gut geeignet, da es eine hohe optische Qualität hinsichtlich hoher Transparent und geringer Trübung aufweist und insbesondere für Spritzen und pharmazeutische Behälter eingesetzt wird. Diese sind insbesondere auch als biokompatible Varianten erhältlich.

Hinsichtlich der Ausbildung einer ebenen Fläche als proximale oder distalen Endfläche 43, 53 können in vorteilhafter Ausführung auch Gießharze eingesetzt werden, welche insbesondere niedrig-viskos sind und gewisse selbst-nivellierende Eigenschaften besitzen.

Alternativ zu einem Extrusionsprozess können die Glasfaser-Bündel oder die Kunststofflichtleiter zu ihrem Schutz auch in einem dünnwandigen Schlauch oder in einem Schrumpfschlauch eingeschlaucht sein. Bei Schrumpfschläuchen können in vorteilhafter Weise extrem dünnwandige Schrumpfschläuche verwendet werden (Beispiel: PET-Schrumpfschlauch mit 6 µm Wandstärke). Denkbar sind auch dünnwandige Netzgewebe-Schläuche aus Glasseide oder Kunststoffseide.

Die Glasfasern können besonders bevorzugt für medizintechnische Anwendungen aus einem Pb- bzw. Schwermetall-freiem Kernglas und Mantelglas bestehen, was insbesondere die RoHS- und REACH-Anforderungen und die medizinische Zulassung begünstigt. Derartige Glassysteme sind zur Herstellung Pb- und schmermetallfreier Fasern, welche seitens der Anmelderin unter dem Namen SCHOTT PURAVIS^{®} bekannt sind, sind u.a. in den Schriften WO 2013/104748 A1 und DE 102007063463 B4 beschrieben. Pb- und schmermetallfreie starre faseroptische Elemente sind in der DE 102013208838 B4 beschrieben. Für Anwendungen im Bereich der Endoskopie eignen sich besonders Glasfasern mit hohen NA-Werten, das heißt mit Akzeptanzwinkeln 2α > 80°, bevorzugt 2α > 100°, um eine breite Ausleuchtung einerseits und eine optimale Lichteinkopplung mittels LEDs andererseits zu ermöglichen. Derartige Fasern sind beispielsweise unter dem Namen SCHOTT PURAVIS^{®} GOF85 oder GOF120 bekannt.

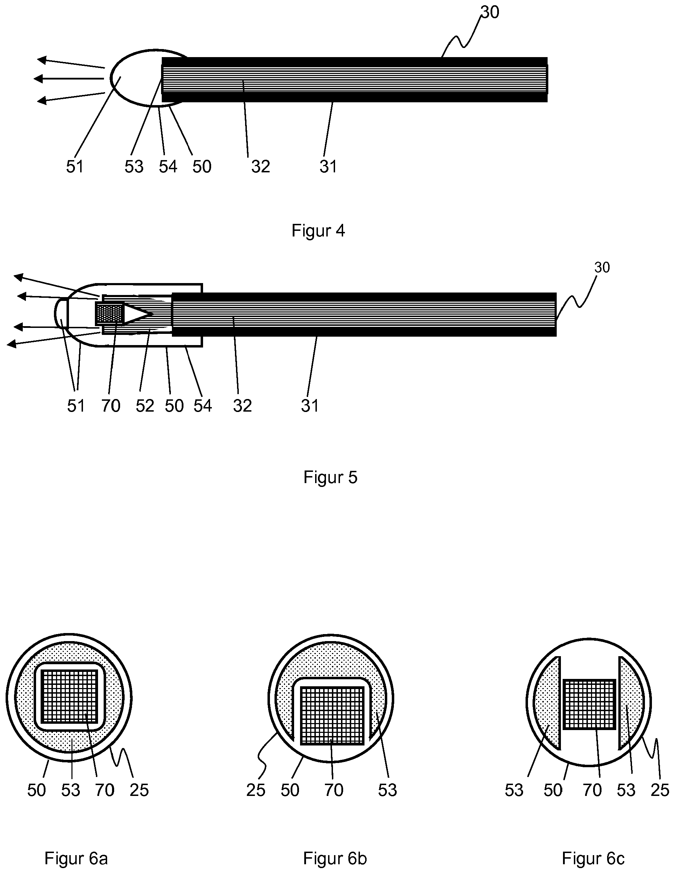

Figur 4 zeigt ausschnittsweise einen alternativen Ansatz eines Beleuchtungslichtleiters 30 mit distaler Hülse 50, was in gleicher Weise auch für die proximale Hülse 40 ausgeführt sein kann.

Hierzu werden, wie schon in Figur 3 beschrieben, die Faserbündel beispielsweise zuvor extrudiert, das heißt das Faserbündel 32 mit einem Kunststoff zu einem Kabel 31 ummantelt, abgelängt und anschließend entweder einem Spritzgieß-Prozess zugeführt, bei dem die Kabelabschnitte direkt mit dem transparent Kunststoff umspritzt werden und dabei eine Hülse, hier eine distale Hülse 50, ausgeprägt wird. Um ein Auffächern der Faserenden zu verhindern muß gegebenenfalls in einem ersten Schritt das Kabelende zumindest an 2 gegenüberliegenden Stellen mittels halbkreisförmigen Spannzangen gepackt werden und zumindest teil-umspritzt werden. In einem 2. Spritzgieß-Prozess kann dann vorgesehen sein, die finale Hülsengeometrie anzuspritzen. Damit ist es möglich, zum einen eine klar transparente Abdeckung der distalen Endfläche 53, gegebenenfalls mit integriert optischer Funktionalität in Form von ausgeformten Linsenelementen (optisches Element 51), sowie eine mit einem anderen Kunststoff, welcher gegebenenfalls ein anderer Kunststoff-Typ und auch opak sein kann, mechanische Schnittstelle 54 auszuprägen. Gleiches gilt für die proximale Hülse 40, bei der mit diesen Verfahrensschritten zum einen eine klar transparente Abdeckung der proximalen Endfläche 43 mit gegebenenfalls angeformten optischen Elementen 41 sowie eine mechanische Schnittstelle 44 herstellbar ist.

Figur 5 zeigt eine Variante der in Figur 3 dargestellten Ausführungsform. Die hier wieder exemplarisch gezeigte distale Hülse 50 am Beleuchtungslichtleiter 30, als extrudiertes Kabel 31 mit dem Faserbündel 32 dargestellt, weist hier zentral einen Bereich auf, indem beispielsweise ein Kamera-Chip 70 (C-MOS-Chip) eingesetzt werden kann, wobei das Faserbündel 32 des Beleuchtungslichtleiters 30 ringförmig, zumindest abschnittsweise ringförmig oder in mindestens zwei Teilsträngen um den Kamera-Chip 70 geführt angeordnet sind. Dazu ist der Aufnahmebereich 52 des Faserbündels 32 entsprechend konisch erweitert. Auch können optische Elemente 51 bei der Herstellung der Hülse angeformt werden, oder können in einem nachgeschalteten Verklebeprozess zusätzlich aufgebracht werden. Damit kann einerseits eine optimale, vor allem schattenfreie Ausleuchtung der zu untersuchenden Gewebe-Fläche und zum anderen eine abbildente Optik für den Kamera-Chip 70 realisiert werden. Ebenso denkbar ist die Integration von sensorischen Elementen, wie Fotodioden oder dgl. zur Detektion von bestimmten Wellenlängen des von der zu untersuchenden Fläche zurückgestreuten Lichtes.

Figur 6a bis 6c zeigen schematisch typische Anordnungen der distalen Endfläche 53 des Beleuchtungslichtleiters 30 in Verbindung mit einem Kamera-Chip 70, wobei in diesen Beispielen die distale Hülse 50 den Abschluß des Schaftes 25 des Endoskops 1 darstellt. Figur 6a zeigt eine Anordnung, bei dem der Kamera-Chip 70 im Wesentlichen von der distalen Endfläche 53 umschlossen ist. Figur 6b zeigt eine im Wesentlichen U-förmig ausgebildeten distalen Endfläche 53. Figur 6c zeigt beispielhaft eine Anordnung, bei der der Kamera-Chip 70 von zwei D-förmig ausgebildeten distalen Endflächen 53 gegenüberliegend umrandet ist. Darüber hinaus sind auch 3- oder 4-geteilte distale Endflächen 53 denkbar, die den Kamera-Chip 70 als kreisförmige oder ovale beziehungsweise nierenförmige Austrittsflächen umschließen.

Die geometrische Anordnung ist dabei entsprechend in der distalen Hülse 50 konstruktiv vorgegeben. Derartige Hülsen lassen sich mittels Spritzgießen besonders kostengünstig herstellen.

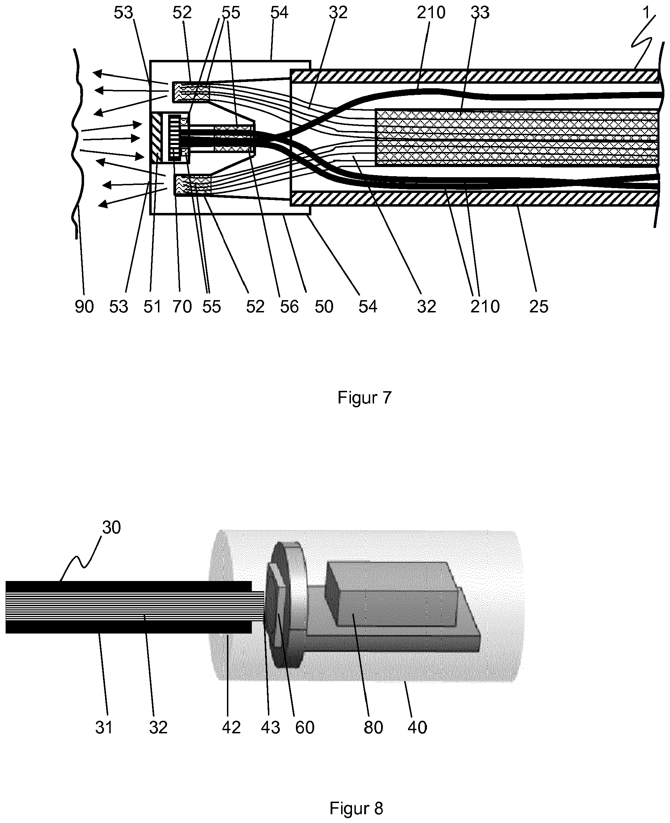

Figur 7 zeigt in der Schnitt-Ansicht beispielsweise eine distale Hülse 50 entsprechend der in Figur 6a gezeigten Anordnung von distaler Endfläche 53 und Kamera-Chip 70.

Beispielhaft ist hier die distale Hülse 50 als Abschluss eines starren Schaftes 25 des Endoskops 1 dargestellt, welcher beispielsweise als Edelstahl-Rohr ausgebildet sein kann. Die distale Endfläche 53 ist hier im Wesentlichen ringförmig um den zentral angeordneten Kamera-Chip 70 angeordnet. Das hiervon abgestrahlte Licht wird von beispielsweise einer zu untersuchenden Gewebeoberfläche 90 reflektiert und von dem Kamera-Chip 70 aufgefangen. Der Kamera-Chip 70 ist zum einen zum Schutz abgedeckt, wobei die Abdeckung als optisches Element 51, zum Beispiel als Sammellinse ausgeführt sein kann. Ebenso denkbar sind mehrlinsige Anordnungen als optisches Element 51. Der Kamera-Chip 70 ist dabei mit elektrischen Leitungen 210 kontaktiert, welche durch eine Durchführung 56 in der distalen Hülse 50 in das Innere des Schaftes 25 geführt sind. Das Faserbündel 32, hier aus Glasfasern mit hoher NA (Akzeptanzwinkel 2α > 100°) ausgeführt, ist hier ringförmig aufgefächert und in einem um die Durchführung 56 angeordneten ringförmigen Aufnahmeabschnitt 52 fixiert. Dieser Aufnahmeabschnitt 52 weist hierbei nahezu zueinander parallel Wandungen auf, um hier möglichst eine parallele Ausrichtung der Fasern zu ermöglichen. An den Aufnahmeabschnitt 52 anschließend weist die distale Hülse 50 konisch ausgeformte Bereiche auf, um das Einfädeln der Fasern zu erleichtern. Das Faserbündel 32 ist im Inneren des Schaftes 25 mit einer Schutzhülle 33 umgeben, welche ein extrudierter Mantel, ein Netzschlauch oder ein Schrumpfschlauch sein kann. Hinsichtlich des nur sehr geringen Bauraums im Schaft 25 ist es besonders vorteilhaft, wenn als Schutzhülle beispielsweise ein dünnwandiger PET-Schrumpfschlauch verwendet wird. Diese weisen eine Wandstärke von < 10 µm auf. Die distale Hülse 50 weist an ihrer Außenkontur ggf. noch weitere mechanische Schnittstellen 54, zum Beispiel in Form von Kragen oder wie gezeigt einen Durchmessersprung auf, um die distale Hülse 50 mit dem Schaft 25 zu verbinden. Weiterhin sind verschiedene Klebebereiche 55 vorgesehen, einerseits zum Fixieren der Fasern des Faserbündels 32 und zum anderen zur Befestigung des Kamera-Chips 70 bzw. zur zusätzlichen Abdichtung der Durchführung 56 der elektrischen Leitungen 210. Besonders vorteilhaft ist es hinsichtlich der Prozesszeiten und damit der Kosten, wenn die gesamte distale Hülse 50 aus einem klar transparenten Kunststoff, zum Beispiel aus PC oder PMMA, ausgeführt ist und als Kleber beziehungsweise Gießharz für die Klebebereiche 55 ein UV-aushärtender Kleber verwendet wird, wobei insbesondere im Aufnahmeabschnitt 52 zur Fixierung der Fasern ein Kleber beziehungsweise ein Gießharz verwendet wird, dessen optischer Brechungsindex im Wesentlichen an den des Kernmaterials der Fasern angepasst und eine Abweichung dieser Brechungsindices maximal ± 0,1, bevorzugt maximal ± 0,05 beträgt und wobei der Brechungsindex der Hülse geringfügig geringer ist als der des Klebers ist.

Eine derartige Ausführung mit den beispielhaft aufgeführten Merkmalen ist natürlich auch für eine proximale Hülse 40 vorstellbar, wobei hier statt des Kamera-Chips 70 eine LED 60 integrierbar sein kann.

In einer hier nicht dargestellten Variante kann auch vorgesehen sein, dass der Kamera-Chip 70 rückseitig in die distale Hülse 50 montiert ist und die distale Endfläche 53 eine Abdeckung ausbildet. Damit kann ohne zusätzliches Abdeckelement eine verbesserte elektrische Isolation erzielt werden.

In Figur 8 ist eine proximale Hülse 40 am Beleuchtungslichtleiter 30 gezeigt, bei der in die proximale Hülse 40 eine Led 60 mit LED-Controller-Einheit 70 integriert ist. Damit kann insbesondere eine platzsparende Lichtquelle realisiert werden. LED 60 und LED-Kontroller-Einheit 70 sind dabei in einer gern. Figur 3 separat hergestellten proximalen Hülse 40 integriert, wobei das Ende des Faserbündels 32 in eine in der proximalen Hülse 40 ausgeprägten Aufnahme 42 montiert bzw. fixiert sind. Die proximale Endfläche 43 kann dabei mit einer klar transparenten Abdeckung versehen sein, die als Kondensor-Linse bzw. den LED-Chip umfassende -struktur ausgeführt sein, um eine optimale Lichteinkopplung in das Faserbündel 32 zu ermöglichen.

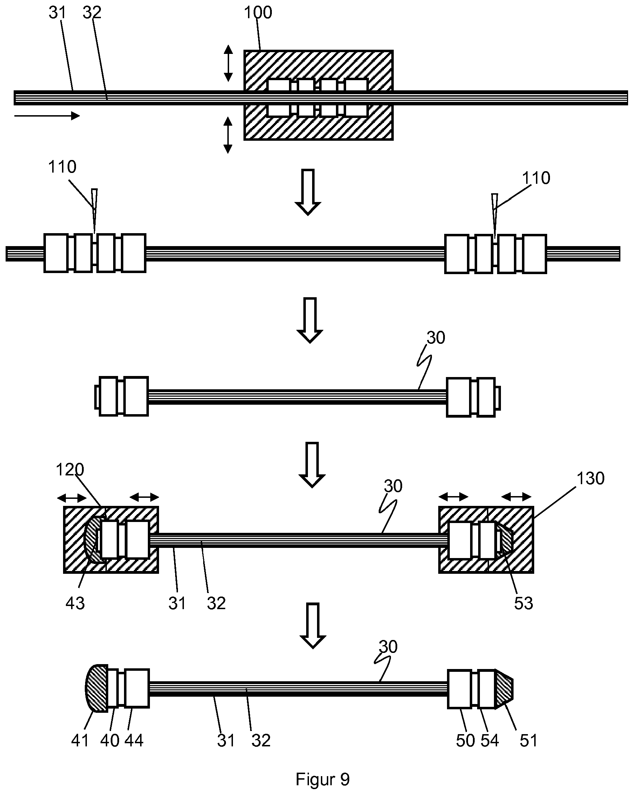

Alternativ kann, wie dies eine stark vereinfachte Prozess-Sequenz in Figur 9 zeigt, in einem "endlos"-Prozess vorgesehen sein, dass ein zuvor extrudiertes Kabel 31 mit dem Faserbündel 32 von einem Abwickler zu einem Aufwickler umgespult wird, und in bestimmten Abständen die Umspulung angehalten und mittels eines 1. Spritzgieß-Werkzeuges 100 eine doppelt ausgeführt Kunststoffhülse aufgespritzt wird. An dieser Stelle wird um das Kabel 31 formschlüssig ohne Zwischenschicht eine Doppel-Hülse aufgeprägt, welche mittels Trennvorrichtungen 110 in einem nachfolgenden Cut-Prozess mit dem Kabel 31 getrennt wird. Dies ist auch direkt nach dem Extrusionsprozess denkbar, insofern dabei ggf. entsprechende Maßnahmen vorgesehen sind die Prozess-Geschwindigkeiten anzupassen beziehungsweise auszugleichen, beispielsweise eine Pufferzone zum Zwischenlagern des extrudierten Kabels. Die so terminierten Kabelabschnitte, welche dem Beleuchtungslichtleiter 30 später entsprechen, können dann in weiteren Schritten mittels einem 2. Und einem 3. Spritzgieß-Werkzeug 120, 130 mit dem finalen Hülsendesign und hier insbesondere mit optisch klar transparenten Kunststoff umspritzt werden, so dass damit dann unter anderem auch einfache Ein- beziehungsweise Austrittsoptiken (optische Elemente 41, 51) an den proximalen bzw. distalen Endflächen 43, 53 des Beleuchtungslichtleiters 30 realisiert werden können. Alternativ kann dies auch in einem Klebe-Prozess realisiert werden, wobei auch weitere Komponenten, unter anderem zum Beispiel auch C-MOS-Kameras oder Sensoren, damit montiert werden können. Der Vorteil liegt hier darin, dass zum einen feste Hülsen herstellbar sind und insbesondere auch die Fixierung im Werkzeug für den 2. finalen Umspritz-Prozess durch eine Ausprägung von entsprechenden mechanischen Schnittstellen 44, 54 erleichtert wird. Damit lassen sich dichte Bündelterminierungen realisieren. Damit lassen sich insbesondere einfache Beleuchtungslichtleiter 30 sehr kostengünstig in hohen Stückzahlen realisieren, was insbesondere für Anwendungen im Disposable-Bereich und aber auch im Konsum-Bereich von hohem Interesse ist.

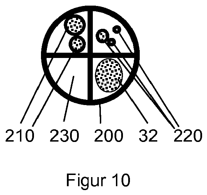

In einer bevorzugten Ausführungsform können sogenannte Multilumen-Kabel 200 hergestellt werden, wie dies Figur 10 schematisch zeigt. Diese können Faserbündel 32, Quarz-Fasern 220, elektrische Kabel 210 sowie einen Fluid-Kanal 230 für eine Medienführung von Gas (zum Beispiel Stickstoff), Wasser, Medikamente bzw. Spülflüssigkeiten aufweisen. Die Quarz-Fasern 220 können dabei bspw. zum optischen Datentransfer bzw. zur Steuerung genutzt werden. Multilumen-Schläuche sind bereits aus der Literatur bekannt. Besonders vorteilhaft ist hier die Integration von Licht- beziehungsweise Energieführenden Komponenten, die hier auf kleinstem Raum eine hohe Funktionalität ermöglichen. Unter anderem kann dabei auch vorgesehen sein, dass die Kabel in einem Koextrusionsprozess gezielt segmentweise transparent oder opak ausgeführt werden und so auch Beleuchtungs- oder optische Detektionsaufgaben erfüllen können.

Eine weitere Alternative zu einer kostengünstigen Terminierung stellen gecrimpte Hülsen dar, wie sie in der DE 102004048741 B3 beschrieben sind. Alternativ dazu können auch Kunststoff-Crimp- bzw. Rasthülsen zum Einsatz kommen, die zuvor im Spritzgussverfahren hergestellt und faltbar mit einem Faltscharnier (hier Film scharnier) ausgeführt sind. Diese Hülsen werden dann am Ende des Kabelabschnitts des extrudierten Kabels rastbar montiert und können dann mit optisch transparentem Kleber ausgegossen beziehungsweise ausgespritzt. Vorteilhaft sind hier auch UV-härtende Kleber. Neben einer Verrastung kann auch vorgesehen sein, dass die Hülsen mittels Laserschweißen, Ultraschallschweißen auf dem Kabel fixiert werden.

Eine weitere Methode ergibt sich aus den elastischen Eigenschaften eines Kabels. Dabei ist vorgesehen, ein extrudiertes Kabel zu schneiden und anschließend den Kabelmantel zu längen und die dabei entstehende Kavität mit optisch klarem Kleber aufzufüllen oder ein vorgefertigtes klar transparentes Kunststoffteil oder einen Lichtleitstab oder Faserstab aus Glas oder Kunststoff in die Kavität einzusetzen und zu fixieren. Zusätzlich kann durch gezieltes Verformen der freiliegenden Kabelabschnitte ein Befestigungselement ausgeformt werden. Insbesondere eigenen sich hier thermoplastische Elastomere (TPE) oder Elastomere, wie Gummi oder Silikon, als Mantelmaterial.

Eine weitere Alternative einer kostengünstigen Terminierung von Lichtleitern kann ein partielles Erhitzen eines mit Gel gefüllten Kabels sein, damit dort das Gel aushärtet und dabei das Kabel geschnitten und ggf. auch umgeformt bzw. Hülsen ausgeformt werden können. Das Kabel kann auch mittels Koextrusion hergestellt sein und einen transparenten Abschnitt entlang der Achse des Kabels aufweisen, über den dann gezielt mittel UV-Licht abschnittsweise, partiell das Gel ausgehärtet werden kann. Hiermit kann ebenfalls ein Endlosprozess für eine Terminierung realisiert werden.

### Bezugszeichenliste:

- 1: Endoskop

- 10: Handstück

- 20: flexibler Abschnitt
- 25: Schaft

- 30: Beleuchtungslichtleiter
- 31: Kabel
- 32: Faserbündel
- 33: Schutzhülle

- 40: proximale Hülse
- 41: optisches Element
- 42: Aufnahmeabschnitt
- 43: proximale Endfläche
- 44: mechanische Schnittstelle

- 50: distale Hülse
- 51: optisches Element
- 52: Aufnahmeabschnitt
- 53: distale Endfläche
- 54: mechanische Schnittstelle
- 55: Klebebereich
- 56: Durchführung

- 60: LED

- 70: Kamera-Chip

- 80: LED-Controller-Einheit

- 90: Gewebeoberfläche

- 100: 1. Spritzgießwerkzeug

- 110: Trennvorrichtung

- 120: 2. Spritzgießwerkzeug

- 130: 3. Spritzgießwerkzeug

- 200: Multilumen-Kabel

- 210: Elektrische Leitungen

- 220: Quarz-Fasern

- 230: Fluid-Kanal

## Patentansprüche

1. Diagnose-, Operations- und/ oder Therapiegerät zur Einbringung in den menschlichen oder tierischen Körper oder zur in-vitro Untersuchung von menschlichen oder tierischen Blutproben oder sonstigen Körperzellen,
insbesondere
Endoskop (1) oder ein Einweg-Endoskop, beinhaltend zumindest einen Beleuchtungslichtleiter (30) und/ oder Bildleiter zur Transmission elektromagnetischer Strahlung, wobei der Beleuchtungslichtleiter oder der Bildleiter jeweils eine proximale Endfläche (43) zur Einbeziehungsweise Auskopplung von elektromagnetischer Strahlung und eine distale Endfläche (53) zur Aus- beziehungsweise Einkopplung elektromagnetischer Strahlung aufweist,
wobei die proximalen und/ oder distalen Endflächen (43, 53) aus zumindest teil- oder abschnittsweise transparenten Kunststoff-Elementen bestehen oder ein transparenter Kunststoff an diese angeformt ist, wobei
der transparente Kunststoff biokompatibel ist und/ oder nicht zytotoxisch wirkt auf menschliche oder tierische Zellkulturen für Einwirkzeiten kleiner einem Tag, und ausgewählt ist aus der Gruppe der Cyclo-Olefin-Co-Polymere, Polycarbonate, Polyethylen-Teraphthalaten, Perfluoralkoxy-Polymere, Polyvinylidenfluoride, Polymethylmethacrylate Polymethylmethacrylimide, Acryl Styrol-Acrylnitril-Co-Polymere oder Raumtemperatur-vernetzenden Silikon, heißvernetzenden Flüssig-Silikone, Epoxid-Gießharze oder -Kleber, thermisch oder UV-vernetzenden Acrylat-Gießharze, Polyurethan-Gießharze, Polyester-Gießharze oder aus Mischungen und/ oder Kombinationen derselben und
der Beleuchtungslichtleiter (30) oder Bildleiter aus einem Faserbündel (32) aus Glasfasern, Quarzfasern oder Kunststoff-Fasern und/ oder aus Einzelfasern aus diesen Materialien besteht, welche zumindest teil- oder abschnittsweise mit einem Mantel umgeben ist und der Mantel aus einem weiteren Kunststoffmaterial besteht und als extrudiertes Kabel (31) ausgeführt ist,
**dadurch gekennzeichnet, dass**
der Mantel (31) aus einem thermoplastischen Elastomer ausgebildet ist.

2. Diagnose-, Operations- und/ oder Therapiegerät nach Anspruch 1, **gekennzeichnet durch** zumindest eines der Merkmale:
- die proximale und/ oder die distale Endfläche (43, 53) des Beleuchtungslichtleiters (30) und/ oder Bildleiters weist zudem jeweils eine mechanische Schnittstelle (44, 54) in Form einer Hülsenkontur auf, welche aus Kunststoff besteht oder mittels Kunststoff-Spritzguss an den Beleuchtungslichtleiter (30) oder Bildleiter angeformt ist, wobei dieser Kunststoff sich vom transparentem Kunststoff der proximalen oder distalen Endfläche zumindest teil- oder abschnittsweise hinsichtlich Material, Transparenz und/ oder Farbe unterscheidet,
- die distale und/ oder proximale Endfläche (43, 53) ist mit der mechanischen Schnittstelle (44, 54) als Hülse ausgeführt, welche separat hergestellt ist, und mittels eines Klebers oder Gießharzes auf das Faserbündelende oder Faserstabende des Beleuchtungslichtleiters (30) oder des Bildleiters fixiert ist, wobei der Kleber als wärmeaushärtender oder UV-Licht-aushärtender Kleber ausgeführt ist, welcher einen optischen Brechungsindex aufweist, der im Wesentlichen dem des Kernmaterials der in den verwendeten Beleuchtungslichtleiter oder Bildleiter verwendeten Fasern oder Faserkomponenten entspricht und die Abweichung dazu maximal ± 0,1, bevorzugt maximal ± 0,05 beträgt und wobei der Brechungsindex der Hülse geringfügig geringer ist als der des Klebers oder des Gießharzes,
- die distale und/ oder proximale Endfläche (43, 53) ist mit der mechanischen Schnittstelle (44, 54) in Form einer Hülse mittels Spritzguss auf zuvor abgelängte Kabelabschnitte angeformt, wobei dieser Prozess als zweistufiger Prozess ausgelegt sein kann, wobei in einem ersten Schritt das Kabelende zumindest an zwei gegenüberliegenden Stellen mittels der Außenkontur des Kabels angepassten Werkzeugen fixiert und zumindest teil- oder abschnittsweise mit einem ersten Kunststoffes umspritzt ist, und in einem zweiten Schritt die Hülsengeometrie mittels eines zweiten Kunststoffes angeformt ist, wobei in einem der Schritte die distale und/ oder proximale Endfläche (43, 53) mit dem transparenten Kunststoff ausformbar ist.

3. Diagnose-, Operations- und/ oder Therapiegerät nach Anspruch 1 oder 2, **dadurch gekennzeichnet,**
**dass** der transparente Kunststoff der proximalen und/ oder distalen Endflächen (43, 53) des Beleuchtungslichtleiters (30) und/ oder Bildleiters eine Oberflächenrauigkeit Ra von ≤ 1,0 µm, vorzugsweise ≤ 0,5 µm, besonders bevorzugt ≤ 0,1 µm aufweist und/ oder
**dass** der transparente Kunststoff der proximalen oder distalen Endflächen (43, 53) des Beleuchtungslichtleiters (30) und/ oder Bildleiters einen Brechungsindex aufweist, der im Wesentlichen dem des Kernmaterials der in dem verwendeten Beleuchtungslichtleiter oder Bildleiter verwendeten Fasern oder Faserkomponenten entspricht und die Abweichung dazu maximal ± 0,1, bevorzugt maximal ± 0,05 beträgt.

4. Diagnose-, Operations- und/ oder Therapiegerät nach einem der Ansprüche 1 bis 3,
**dadurch gekennzeichnet,**
**dass** der Mantel aus einem zumindest teil- oder abschnittsweise transluzenten, opaken oder eingefärbten Kunststoff besteht.

5. Diagnose-, Operations- und/ oder Therapiegerät nach einem der Ansprüche 1 bis 4,
**dadurch gekennzeichnet,**
**dass** der Beleuchtungslichtleiter (30) oder Bildleiter
- aus flexiblen oder semi-flexiblen Faserbündeln besteht und der Mantel zumindest teil- oder abschnittsweise als starre Hülle ausgeführt ist, oder
- aus gezogenen Faserstäben oder gepressten Faserstäben besteht und einen starren Beleuchtungslichtleiter (30) oder Bildleiter ausbildet.

6. Diagnose-, Operations- und/ oder Therapiegerät nach zumindest einem der Ansprüche 2 bis 5,
**dadurch gekennzeichnet,**
**dass** die Fasern, Faserbündel, Faserstäbe oder gepressten Faserstäbe
aus einem Pb- bzw. Schwermetall-freiem Kernglas und Mantelglas bestehen und/ oder aus einem Glassystem bestehen, welches für das zu leitende Licht einen Akzeptanzwinkel 2α von größer 80°, besonders bevorzugt von größer 100° aufweist.

7. Diagnose-, Operations- und/ oder Therapiegerät nach Anspruch 2,
**dadurch gekennzeichnet,**
**dass** die Hülse Aufnahmeabschnitte (42, 52) zur Aufnahme eines Faserbündels (32) aufweist, die von einem zunächst konischen Abschnitt in einen Abschnitt münden, der im Wesentlichen parallel angeordnete Seitenwände aufweist und die Hülse weiterhin Aufnahmen für elektronische Komponenten aufweist, und diese Aufnahmenabschnitte (42, 52) den Bereich der Aufnahme für elektronische Komponenten zumindest bereichsweise umschließen.

8. Diagnose-, Operations- und/ oder Therapiegerät nach einem der Ansprüche 1 bis 7,
**dadurch gekennzeichnet,**
**dass** in einem Endlos-Prozess auf ein zuvor extrudiertes Kabel (31) in bestimmten Abständen entsprechend der finalen Bauteillänge eine hinsichtlich ihrer Kontur doppelten Hülse als mechanische Schnittstelle (44, 54) angeformt ist, welche dann in einem nächsten Prozessschritt trennbar ist, in die damit erzeugten Kabelabschnitte mittels ein oder mehreren weiteren
Spritzgieß-Prozessen mit klar transparenten Kunststoff die proximalen und/ oder distalen Endflächen (43, 53) anformbar sind.

9. Diagnose-, Operations- und/ oder Therapiegerät nach einem der Ansprüche 1 bis 7,
**dadurch gekennzeichnet,**
**dass** ein extrudiertes Kabel (31) in bestimmten Abständen oder ein entsprechender Faserbündelabschnitt, welcher mit einem Schlauch oder Schrumpfschlauch umgeben ist, entsprechend der finalen Bauteillänge geteilt ist und die im inneren des extrudierten Kabelabschnitts oder Faserbündelabschnitts vorhandenen Faserbündel (32) nach innen verschoben sind und der Raum zwischen Faserbündelende und Mantelrand oder Rand des Schlauchs oder Schrumpfschlauches mit einem transparenten selbst nivellierendem Kunststoff gefüllt ist.

10. Diagnose-, Operations- und/ oder Therapiegerät nach einem der Ansprüche 1 bis 7,
**dadurch gekennzeichnet,**
**dass** ein zuvor extrudiertes Kabel (31) in bestimmten Abständen oder ein entsprechender Faserbündelabschnitt, welcher mit einem Schlauch oder Schrumpfschlauch umgeben ist, entsprechend der finalen Bauteillänge geteilt ist und der Kabelmantel, der Schlauch oder Schrumpfschlauch gegenüber dem Faserbündel gelängt und die dabei entstehende Kavität mit optisch transparenten Kunststoff aufgefüllt ist oder ein vorgefertigtes klar transparentes Kunststoffteil oder ein Lichtleitstab oder Faserstab aus Glas oder Kunststoff in die Kavität eingesetzt und fixiert ist.

11. Diagnose-, Operations- und/ oder Therapiegerät nach einem der Ansprüche 8 oder 10,
**dadurch gekennzeichnet,**
**dass** der Mantelabschnitt, Schlauch- oder Schrumpfschlauchabschnitt, der die Kavität ausbildet, verformt ist und eine bestimmte Lichteintritts- oder Lichtaustrittskontur nach Aushärten des Kunststoffes oder nach Einsetzen des Kunststoffteils oder des Lichtleitstabs ausbildet.

12. Diagnose-, Operations- und/ oder Therapiegerät nach einem der Ansprüche 1 bis 11,
**gekennzeichnet durch** zumindest eines der folgenden Merkmale:
- die proximalen oder distalen Endflächen (43, 53) weisen weitere aktive elektronische Elemente in Form von LEDs (60), Laser-Dioden, Sensoren oder Kamera-Chips (70) auf, welche in die angeformten Hülsen integrierbar oder an diese mittels einer Rastverbindung ansteckbar sind,
- zusätzliche Elemente aus Glas oder Kunststoff zur Abdeckung der aktiven elektronischen Elemente an den proximalen oder distalen Endflächen (43, 53) vorgesehen sind,
- die proximalen und/ oder distalen Endflächen (43, 53) sind als optisches Element zur Erzielung einer bestimmten Strahlformung ausgeführt, und weisen dabei eine plane, konvexe, konkave Fläche oder eine hinsichtlich ihrer Topographie beliebig gestaltete Freiformfläche auf.

13. Diagnose-, Operations- und/ oder Therapiegerät nach einem der Ansprüche 1 bis 12,
**dadurch gekennzeichnet,**
**dass** das extrudierte Kabel (31) für den Beleuchtungslichtleiter (30) oder Bildleiter als Hybrid-Kabel oder Multilumen-Kabel (200) ausgeführt ist, und vorzugsweise in einem Koextrusionsprozess segmentweise transparent oder opak ausgeführt ist.

14. Diagnose-, Operations- und/ oder Therapiegerät nach einem der Ansprüche 1 bis 13,
**dadurch gekennzeichnet,**
**dass** das Hybrid-Kabel als Multilumen-Kabel (200) ausgeführt ist, mit dem Faserbündel (32), einzelne Quarz-Fasern (220), Medien in Form von Gasen oder Flüssigkeiten in einem Fluid-Kanal (230) und/ oder elektrische Leitungen (210) getrennt führbar sind.

15. Diagnose-, Operations- und/ oder Therapiegerät nach Anspruch 14,
**dadurch gekennzeichnet,**
**dass** das Multilumen-Kabel (200) einen flexiblen Abschnitt (20) des Endoskops (1) oder das Multilumen-Kabel (200) aus einem bei Raumtemperatur starren Kunststoff besteht und einen starren Schaft (25) des Endoskops (1) ausbildet.

16. Verwendung der Beleuchtungslichtleiter (30) und/ oder Bildleiter nach mindestens einem der vorstehenden Ansprüche in In-Vitro-Diagnostik-Geräten.

## Claims

1. A diagnostic, surgical and/or therapeutic device for being introduced into the human or animal body or for in-vitro examination of human or animal blood samples or other body cells;
in particular an endoscope (1) or single-use endoscope comprising at least one illumination light guide (30) and/or image guide for transmitting electromagnetic radiation, wherein said illumination light guide or image guide has a proximal end face (43) for injection or emission of electromagnetic radiation and a distal end face (53) for emission or injection of electromagnetic radiation;
wherein said proximal and/or distal end faces (43, 53) consist of elements made of plastics material, which are transparent at least partially or in sections thereof, or have a transparent plastics material overmoulded thereon;
wherein said transparent plastics material is biocompatible and/or non-cytotoxic to human or animal cell cultures over exposure durations of less than one day, and is selected from the group consisting of cyclic olefin copolymers, polycarbonates, polyethylene terephthalates, perfluoroalkoxy polymers, polyvinylidene fluorides, polymethyl methacrylates, polymethyl methacrylimides, acrylic styrene acrylonitrile copolymers, or room temperature crosslinking silicone, hot crosslinking liquid silicones, epoxy casting resins or adhesives, thermally or UV crosslinking acrylate casting resins, polyurethane casting resins, polyester casting resins, or mixtures and/or combinations thereof; and
wherein the illumination light guide (30) or image guide is made up of a fibre bundle (32) consisting of glass optical fibres, quartz optical fibres, or plastic optical fibres, and/or comprises individual fibres made of these materials, which are enclosed at least partially or in sections thereof by a jacket, and wherein the jacket is made of a further plastics material, and is provided in the form of an extruded cable (31);
**characterised in that**
the jacket (31) is made from a thermoplastic elastomer.

2. The diagnostic, surgical and/or therapeutic device according to claim 1, **characterised by** at least one of the following features:
- the proximal and/or distal end faces (43, 53) of the illumination light guide (30) and/or image guide furthermore have a mechanical interface (44, 54) in the form of a sleeve contour which is made of plastics material or is injection moulded from plastics material onto the illumination light guide (30) or image guide, wherein this plastics material differs from the transparent plastics material of the proximal or distal end faces at least partially or in sections thereof in terms of its material, transparency, and/or colour;
- the distal and/or proximal end faces (43, 53) is provided with the mechanical interface (44, 54) in the form of a sleeve which is produced separately and is fixed on the fibre bundle end or fibre rod end of the illumination light guide (30) or image guide using an adhesive or casting resin, wherein the adhesive is in the form of a thermally curing or UV light curing adhesive which has an optical refractive index substantially matching that of the core material of the fibres or fibre components used in the illumination light guide or image guide, with a deviation thereto of not more than ± 0.1, preferably not more than ± 0.05, and wherein the refractive index of the sleeve is slightly lower than that of the adhesive or casting resin;
- the distal and/or proximal end faces (43, 53) is provided with the mechanical interface (44, 54) in the form of a sleeve which is made by injection moulding onto cable sections previously cut to length, wherein this process can be implemented as a two-stage process, wherein in a first step the cable end is fixed at least at two opposite points by tools which are adapted to the outer contour of the cable, and is overmoulded at least partially or in sections thereof with a first plastics material, and in a second step the sleeve geometry is moulded thereto using a second plastics material, wherein the distal and/or proximal end faces (43, 53) can be moulded in any one of these steps using the transparent plastics material.

3. The diagnostic, surgical and/or therapeutic device according to any one of claims 1 or 2, **characterised in that**
the transparent plastics material of the proximal and/or distal end faces (43, 53) of the illumination light guide (30) and/or image guide exhibits a surface roughness Rₐ of < 1.0 µm, preferably ≤ 0.5 µm, most preferably ≤ 0.1 µm; and/or
wherein the transparent plastics material of the proximal or distal end faces (43, 53) of the illumination light guide (30) and/or image guide has a refractive index which substantially matches that of the core material of the fibres or fibre components used in the illumination light guide or image guide, with a deviation thereto of not more than ± 0.1, preferably not more than ± 0.05.

4. The diagnostic, surgical and/or therapeutic device according to any one of claims 1 to 3, **characterised in that**
the jacket is made of a plastics material that is translucent, opaque, or dyed at least partially or in sections thereof.

5. The diagnostic, surgical and/or therapeutic device according to any one of claims 1 to 4, **characterised in that** the illumination light guide (30) or image guide
- consists of flexible or semi-flexible fibre bundles, and the jacket is in the form of a rigid sheath at least partially or in sections thereof; or
- consists of drawn fibre rods or pressed fibre rods and forms a rigid illumination light guide (30) or image guide.

6. The diagnostic, surgical and/or therapeutic device according to any one of claims 2 to 5, **characterised in that**
the fibres, fibre bundles, fibre rods, or pressed fibre rods are made of a Pb-free or heavy metal-free core glass and cladding glass; and/or are made of a glass system which has an acceptance angle 2α of greater than 80°, most preferably greater than 100° for the light to be carried.

7. The diagnostic, surgical and/or therapeutic device according to claim 2, **characterised in that**
the sleeve has receptacle areas (42, 52) for accommodating a fibre bundle (32), which receptacle areas comprise an initially conical portion transitioning into a portion that has substantially parallel side walls, and wherein the sleeve furthermore has seats for electronic components, and wherein said receptacle areas (42, 52) at least partially surround the area of the seat for electronic components.

8. The diagnostic, surgical and/or therapeutic device according to any one of claims 1 to 7, **characterised in that**
in a continuous process, a double contour sleeve is overmoulded on a previously extruded cable (31) at specific intervals corresponding to the length of the final component to define the mechanical interface (44, 54) which can then be severed in a next process step, and the proximal and/or distal end faces (43, 53) can be moulded to the so produced cable sections in one or more further injection moulding processes using clear transparent plastics material.

9. The diagnostic, surgical and/or therapeutic device according to any one of claims 1 to 7, **characterised in that**
an extruded cable (31) is divided at specific intervals, or a corresponding fibre bundle section enclosed by a tube or shrink tube is divided according to the length of the final component, and the fibre bundles (32) disposed inside the extruded cable section or fibre bundle section are offset inwards, and the space between the fibre bundle end and the edge of the jacket or edge of the tube or shrink tube is filled with a transparent self-levelling plastics material.

10. The diagnostic, surgical and/or therapeutic device according to any one of claims 1 to 7, **characterised in that**
a previously extruded cable (31) is divided at specific intervals, or a corresponding fibre bundle section enclosed by a tube or shrink tube is divided according to the length of the final component, and the cable jacket, the tube, or the shrink tube is elongated relative to the fibre bundle, and the resulting cavity is filled with an optically transparent plastics material, or a prefabricated clear transparent plastics part or a light guiding rod or fibre rod made of glass or plastics material is inserted and fixed in the cavity.

11. The diagnostic, surgical and/or therapeutic device according to any one of claims 8 or 10, **characterised in that**
the jacket section, tube section or shrink tube section defining the cavity is reshaped and forms a specific light entry contour or light exit contour once the plastics material has been cured or once the plastics part or light guiding rod has been inserted.

12. The diagnostic, surgical and/or therapeutic device according to any one of claims 1 to 11, **characterised by** at least one of the following features:
- the proximal or distal end faces (43, 53) comprise further active electronic components in the form of LEDs (60), laser diodes, sensors, or camera chips (70) which can be integrated into the overmoulded sleeves or can be fitted thereto through a snap-in connection;
- additional elements made of glass or plastics material are provided on the proximal or distal end faces (43, 53) for covering the active electronic components;
- the proximal and/or distal end faces (43, 53) are in the form of an optical element for achieving a specific type of beam shaping and have a planar or convex or concave surface or a free-form surface of any desired topography.

13. The diagnostic, surgical and/or therapeutic device according to any one of claims 1 to 12, **characterised in that**
the extruded cable (31) for the illumination light guide (30) or image guide is in the form of a hybrid cable or a multi-lumen cable (200), and is preferably produced by a co-extrusion process so as to be transparent or opaque in segments thereof.

14. The diagnostic, surgical and/or therapeutic device according to any one of claims 1 to 13, **characterised in that**
the hybrid cable is in the form of a multi-lumen cable (200) that allows to separately route fibre bundles (32), individual quartz fibres (220), media in the form of gases or liquids in a fluid passage (230), and/or electrical wires (210).

15. The diagnostic, surgical and/or therapeutic device according to claim 14, **characterised in that**
the multi-lumen cable (200) defines a flexible portion (20) of the endoscope (1), or the multi-lumen cable (200) is made of a plastics material that is rigid at room temperature and defines a rigid shaft (25) of the endoscope (1).

16. Use of the illumination light guide (30) and/or image guide according to at least one of the preceding claims for in-vitro diagnostic devices.

## Revendications

1. Dispositif de diagnostic, d'opération et/ou de thérapie, destiné à être introduit dans le corps humain ou animal ou à effectuer des examens in vitro d'échantillons sanguins humains ou animaux ou d'autres cellules du corps,
en particulier
endoscope (1) ou endoscope à usage unique, comprenant au moins un guide de lumière d'éclairage (30) et/ou un guide d'images pour la transmission d'un rayonnement électromagnétique, le guide de lumière d'éclairage ou le guide d'images présentant respectivement une face d'extrémité proximale (43), destinée au couplage entrant ou sortant d'un rayonnement électromagnétique, et une face d'extrémité distale (53) destinée au couplage sortant ou entrant d'un rayonnement électromagnétique,
les faces d'extrémité proximales et/ou distales (43, 53) étant constituées d'éléments en matière plastique qui sont transparents au moins en partie ou par portions, ou une matière plastique transparente étant formée sur celles-ci, la matière plastique transparente étant biocompatible et/ou n'ayant pas d'action cytotoxique sur des cultures cellulaires humaines ou animales, pendant des temps d'action inférieurs à une journée, et étant choisie dans le groupe des copolymères d'oléfines cycliques, polycarbonates, polyéthylène téréphtalates, polymères perfluoroalkoxy, fluorures de polyvinylidène, polyméthacrylates de méthyle, polyméthacrylimides de méthyle, copolymères styrène-nitrile acryliques ou silicone à réticulation à température ambiante, silicone à réticulation à chaud, silicones liquides, résines de coulée ou adhésifs époxy, résines acrylates de coulée à réticulation thermique ou UV, résines polyuréthanes de coulée, résines polyesters de coulée, ou de mélanges et/ou de combinaisons de ceux-ci, et
le guide de lumière d'éclairage (30) ou le guide d'images étant constitué d'un faisceau de fibres (32) formé de fibres de verre, fibres en quartz ou fibres en matière plastique et/ou de fibres individuelles faites de ces matériaux, qui est entouré d'une gaine au moins en partie ou par portions, et la gaine étant constituée d'un autre matériau plastique et étant réalisée sous forme de câble (31) extrudé,
**caractérisé en ce que** la gaine (31) est réalisée à partir d'un élastomère thermoplastique.

2. Dispositif de diagnostic, d'opération et/ou de thérapie selon la revendication 1, **caractérisé en ce qu'**il présente au moins une des caractéristiques suivantes :
- la face d'extrémité proximale et/ou distale (43, 53) du guide de lumière d'éclairage (30) et/ou du guide d'images présente en outre respectivement une interface mécanique (44, 54) sous la forme d'un contour de manchon qui est constitué de matière plastique ou est formé par moulage par injection de matière plastique sur le guide de lumière d'éclairage (30) ou le guide d'images, ladite matière plastique étant différente, au moins en partie ou par portions, de la matière plastique transparente de la face d'extrémité proximale ou distale, en ce qui concerne le matériau, la transparence et/ou la couleur,
- la face d'extrémité distale et/ou proximale (43, 53) est réalisée avec l'interface mécanique (44, 54) sous forme de manchon qui est fabriqué séparément et est fixé à l'aide d'un adhésif ou de résine coulée à l'extrémité du faisceau de fibres ou l'extrémité de la tige de fibres du guide de lumière d'éclairage (30) ou du guide d'images, l'adhésif étant réalisé sous forme d'adhésif durcissant à la chaleur ou à la lumière UV qui présente un indice de réfraction optique correspondant sensiblement à celui du matériau du coeur des fibres ou des composants de fibres utilisés dans le guide de lumière d'éclairage ou le guide d'images utilisés, et l'écart par rapport à celui-ci étant au maximum de + 0,1, de préférence au maximum de + 0,05, et où l'indice de réfraction du manchon est légèrement inférieur à celui de l'adhésif ou de la résine coulée,
- la face d'extrémité distale et/ou proximale (43, 53) est réalisée avec l'interface mécanique (44, 54) sous la forme d'un manchon, par moulage par injection sur des tronçons de câble préalablement coupés à longueur, ce processus pouvant être conçu en tant que processus à deux étapes où, dans une première étape, l'extrémité du câble est fixée à au moins deux emplacements opposés, au moyen d'outils adaptés au contour extérieur du câble, et est entourée, au moins en partie ou par portions, par injection avec une première matière plastique, et où, dans une deuxième étape, la géométrie du manchon est façonnée à l'aide d'une deuxième matière plastique, sachant que dans une des étapes, la face d'extrémité distale et/ou proximale (43, 53) peut être formée avec la matière plastique transparente.

3. Dispositif de diagnostic, d'opération et/ou de thérapie selon la revendication 1 ou 2, **caractérisé**
**en ce que** la matière plastique transparente des faces d'extrémité proximales et/ou distales (43, 53) du guide de lumière d'éclairage (30) et/ou du guide d'images présente une rugosité de surface Ra < 1,0 µm, de préférence < 0,5 µm, et de manière particulièrement avantageuse < 0,1 µm, et/ou
**en ce que** la matière plastique transparente des faces d'extrémité proximales ou distales (43, 53) du guide de lumière d'éclairage (30) et/ou du guide d'images présente un indice de réfraction qui correspond sensiblement à celui du matériau du coeur des fibres ou des composants de fibres utilisés dans le guide de lumière d'éclairage ou le guide d'images utilisés, et l'écart par rapport à celui-ci est au maximum de + 0,1, de préférence au maximum de + 0,05.

4. Dispositif de diagnostic, d'opération et/ou de thérapie selon une des revendications 1 à 3,
**caractérisé en ce que** la gaine est constituée d'une matière plastique qui est translucide, opaque ou teintée, au moins en partie ou par portions.

5. Dispositif de diagnostic, d'opération et/ou de thérapie selon une des revendications 1 à 4,
**caractérisé en ce que** le guide de lumière d'éclairage (30) ou le guide d'images
- est constitué de faisceaux de fibres flexibles ou semi-flexibles, et la gaine est réalisée au moins en partie ou par portions sous forme de manchon rigide, ou
- est constitué de tiges de fibres étirées ou de tiges de fibres comprimées et forme un guide de lumière d'éclairage (30) ou un guide d'images rigides.

6. Dispositif de diagnostic, d'opération et/ou de thérapie selon au moins une des revendications 2 à 5,
**caractérisé en ce que** les fibres, faisceaux de fibres, tiges de fibres ou tiges de fibres comprimées sont constitués d'un verre de coeur et d'un verre de gaine exempt de Pb et de métaux lourds, ou d'un système de verre qui présente pour la lumière à guider, un angle d'acceptation 2α supérieur à 80°, de manière particulièrement avantageuse supérieur à 100°.

7. Dispositif de diagnostic, d'opération et/ou de thérapie selon la revendication 2,
**caractérisé en ce que** le manchon présente des parties (42, 52) destinées à accueillir un faisceau de fibres (32) qui, depuis une partie d'abord conique, débouchent dans une partie présentant des parois latérales disposées de manière sensiblement parallèle, et le manchon présente en outre des parties accueillant des composants électroniques, et lesdites parties d'accueil (42, 52) entourent au moins par portions la zone prévue pour l'accueil de composants électroniques.

8. Dispositif de diagnostic, d'opération et/ou de thérapie selon une des revendications 1 à 7,
**caractérisé en ce que** dans un processus sans fin, un manchon, qui est double en ce qui concerne son contour, est formé en tant qu'interface mécanique (44, 54) sur un câble (31) préalablement extrudé, à des distances définies en fonction de la longueur finale du composant, manchon qui peut ensuite être séparé lors d'une étape de processus subséquente, les faces d'extrémité proximales et/ou distales (43, 53) pouvant être formées dans les tronçons de câble ainsi réalisés, à l'aide d'un ou plusieurs autres processus de moulage par injection avec de la matière plastique transparente claire.

9. Dispositif de diagnostic, d'opération et/ou de thérapie selon une des revendications 1 à 7,
**caractérisé en ce qu'**un câble (31) extrudé est divisé à intervalles définis, ou un tronçon de faisceau de fibres correspondant, qui est entouré d'un tube ou d'un tube thermorétractable, est divisé en fonction de la longueur finale du composant, et les faisceaux de fibres (32) présents à l'intérieur du tronçon de câble ou du tronçon de faisceau de fibres extrudés sont glissés vers l'intérieur, et l'espace entre l'extrémité du faisceau de fibres et le bord de la gaine ou le bord du tube ou du tube thermorétractable est rempli avec une matière plastique transparente autonivelante.

10. Dispositif de diagnostic, d'opération et/ou de thérapie selon une des revendications 1 à 7,
**caractérisé en ce qu'**un câble (31) préalablement extrudé est divisé à intervalles définis, ou un tronçon de faisceau de fibres correspondant, qui est entouré d'un tube ou d'un tube thermorétractable, est divisé en fonction de la longueur finale du composant, et la gaine de câble, le tube ou le tube thermorétractable sont coupés à longueur par rapport au faisceau de fibres, et la cavité ainsi créée est remplie avec de la matière plastique optiquement transparente, ou un élément préfabriqué en matière plastique transparente claire ou une tige de guide de lumière ou une tige de fibres en verre ou matière plastique est mis(e) en place dans la cavité et est fixé(e).

11. Dispositif de diagnostic, d'opération et/ou de thérapie selon une des revendications 8 ou 10,
**caractérisé en ce que** le tronçon de gaine, le tronçon de tube ou de tube thermorétractable, qui forme la cavité, est déformé et forme un contour défini d'entrée de lumière ou de sortie de lumière, après durcissement de la matière plastique ou après mise en place de l'élément en matière plastique ou de la tige de guide de lumière.

12. Dispositif de diagnostic, d'opération et/ou de thérapie selon une des revendications 1 à 11,
**caractérisé en ce qu'**il présente au moins une des caractéristiques suivantes :
- les faces d'extrémité proximales ou distales (43, 53) présentent d'autres éléments électroniques actifs sous forme de LEDs (60), diodes laser, capteurs ou puces de caméra (70), qui peuvent être intégrés dans les manchons rapportés ou être raccordés à ceux-ci à l'aide d'une liaison par encliquetage,
- des éléments supplémentaires en verre ou matière plastique en vue du recouvrement des éléments électroniques actifs sur les faces d'extrémité proximales ou distales (43, 53),
- les faces d'extrémité proximales et/ou distales (43, 53) sont réalisées sous forme d'élément optique, en vue d'obtenir une mise en forme précise du rayon, et présentent alors une surface plane, convexe ou concave ou une surface de forme libre réalisée de n'importe quelle manière en ce qui concerne sa topographie.

13. Dispositif de diagnostic, d'opération et/ou de thérapie selon une des revendications 1 à 12,
**caractérisé en ce que** le câble (31) extrudé pour le guide de lumière d'éclairage (30) ou le guide d'images est réalisé comme câble hybride ou câble à lumières multiples (200) et est de préférence réalisé de manière transparente ou opaque par segments, dans un processus de coextrusion.

14. Dispositif de diagnostic, d'opération et/ou de thérapie selon une des revendications 1 à 13,
**caractérisé en ce que** le câble hybride est réalisé comme câble à lumières multiples (200) qui permet de guider séparément des faisceaux de fibres (32), des fibres en quartz (220) individuelles, des fluides sous forme de gaz ou de liquides dans un conduit de fluide (230) et/ou des lignes électriques (210).

15. Dispositif de diagnostic, d'opération et/ou de thérapie selon la revendication 14,
**caractérisé en ce que** le câble à lumières multiples (200) forme une partie flexible (20) de l'endoscope (1) ou le câble à lumières multiples (200) est constitué d'une matière plastique rigide à la température ambiante et forme un fût (25) rigide de l'endoscope (1).

16. Utilisation des guides de lumière d'éclairage (30) et/ou des guides d'images selon au moins une des revendications précédentes, dans des dispositifs de diagnostic in vitro.
